# FASCICULE DE BREVET EUROPEEN

(11) **EP 3 292 145 B1**
(45) Date de publication et mention de la délivrance du brevet: **08.01.2025**
(21) Numéro de dépôt: 16726133.8
(22) Date de dépôt: 06.05.2016
(51) Int. Cl.: C07K 16/00

(54) **MUTANTS FC A ACTIVITÉ FONCTIONNELLE MODIFIÉE**
FC-MUTANTEN MIT MODIFIZIERTER FUNKTIONELLER AKTIVITÄT
FC MUTANTS WITH MODIFIED FUNCTIONAL ACTIVITY

(30) Priorité: 07.05.2015 FR 1554101
(43) Date de publication de la demande: 14.03.2018
(73) Titulaire: Laboratoire Français du Fractionnement et des Biotechnologies, 92800 Puteaux (FR)
(72) Inventeur: MONNET, Céline, 59130 Lambersart (FR); MONDON, Philippe, 62840 Neuve Chapelle (FR); FONTAYNE, Alexandre, 59110 La Madeleine (FR); DE ROMEUF, Christophe, 59130 Lambersart (FR)
(74) Mandataire: Lavoix
(86) Numéro de dépôt international: PCT/FR2016/051068
(87) Numéro de publication internationale: WO 2016/177984

(56) Documents cités:
- WO-A1-2005/040221
- WO-A1-2005/040221
- WO-A1-2005/040221
- WO-A1-2011/044368
- WO-A1-2011/044368
- WO-A2-2010/106180
- WO-A2-2010/106180
- WO-A2-2010/106180
- FR-A1- 3 012 453
- FR-A1- 3 012 453
- US-A1- 2014 342 404
- US-A1- 2014 342 404
- US-A1- 2014 342 404
- SHIELDS R L ET AL: "High resolution mapping of the binding site on human IgG1 for FcgammaRI, FcgammaRII, FcgammaRIII, and FcRn and design of IgG1 variants with improved binding to the FcgammaR", JOURNAL OF BIOLOGICAL CHEMISTRY,, vol. 276, no. 9, 2 March 2001 (2001-03-02), pages 6591 - 6604, XP002271092, ISSN: 0021-9258, DOI: 10.1074/JBC.M009483200
- SHIELDS R L ET AL: "High resolution mapping of the binding site on human IgG1 for FcgammaRI, FcgammaRII, FcgammaRIII, and FcRn and design of IgG1 variants with improved binding to the FcgammaR", JOURNAL OF BIOLOGICAL CHEMISTRY,, vol. 276, no. 9, 2 March 2001 (2001-03-02), pages 6591 - 6604, XP002271092, ISSN: 0021-9258, DOI: 10.1074/JBC.M009483200
- SHIELDS R L ET AL: "High resolution mapping of the binding site on human IgG1 for FcgammaRI, FcgammaRII, FcgammaRIII, and FcRn and design of IgG1 variants with improved binding to the FcgammaR", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02), pages 6591 - 6604, XP002271092, ISSN: 0021-9258, DOI: 10.1074/JBC.M009483200

## Description

La présente invention se rapporte à un polypeptide comprenant une région Fc mutée et présentant une activité fonctionnelle médiée par la région Fc modifiée par rapport à un polypeptide parent.

Un anticorps est constitué d'un tétramère de chaînes lourdes et légères. Les deux chaînes légères sont identiques entre elles, et les deux chaînes lourdes sont identiques et reliées par des ponts disulfures. Il existe cinq types de chaînes lourdes (alpha, gamma, delta, epsilon, mu), qui déterminent les classes d'immunoglobulines (IgA, IgG, IgD, IgE, IgM). Le groupe de la chaîne légère comprend deux sous-types, lambda et kappa.

Les IgG sont des anticorps solubles qui peuvent être trouvés dans le sang et d'autres fluides corporels. L'IgG est une glycoprotéine en forme de Y avec un poids moléculaire approximatif de 150 kDa, composée de deux chaînes lourdes et deux chaînes légères. Chaque chaîne se distingue en une région constante et une région variable. Les deux domaines carboxy-terminaux des chaînes lourdes forment le fragment Fc, tandis que les domaines amino-terminaux des chaînes lourdes et légères reconnaissent l'antigène et sont nommés fragment Fab.

Les protéines de fusion Fc sont créées par une combinaison d'un fragment Fc d'anticorps avec un domaine protéique qui fournit la spécificité pour une cible thérapeutique donnée. Des exemples sont des combinaisons du fragment Fc avec tout type de protéines thérapeutiques ou leurs fragments.

Les anticorps thérapeutiques et les protéines de fusion Fc sont utilisés aujourd'hui pour traiter diverses maladies, comme la polyarthrite rhumatoïde, le psoriasis, la sclérose en plaques et de nombreuses formes de cancer. Des anticorps thérapeutiques peuvent être des anticorps monoclonaux ou polyclonaux. Les anticorps monoclonaux sont obtenus à partir d'une lignée cellulaire de production d'anticorps unique, qui montre une spécificité identique pour un seul antigène. Les protéines de fusion Fc thérapeutiques sont utilisées ou développées comme médicaments contre les maladies autoimmunes, comme l'étanercept (Enbrel d'Amgen, qui est un récepteur TNF lié à un fragment Fc) ou Alefacept (Amevive de Biogen Idée, qui est LFA-3 lié à la portion Fc d'IgG1 humaine).

Pour que les anticorps et protéines de fusion Fc puissent exercer leurs effets, il est nécessaire que le fragment Fc présente la meilleure activité possible (par exemple cytotoxicité cellulaire dépendante des anticorps, cytotoxicité dépendante du complément ou phagocytose cellulaire dépendante des anticorps), i.e. qu'il soit optimisé. Une telle optimisation permettrait d'obtenir une protéine ayant une activité et/ou une efficacité améliorée(s) et/ou des effets secondaires diminués.

WO2010/106180 décrit des variants Fc comprenant des mutations.

La Demanderesse a maintenant mis au point des fragments Fc particuliers, présentant une activité améliorée. Ces fragments peuvent être utilisables en thérapie, en vue d'apporter une meilleure efficacité au produit qui les contient.

La présente invention fournit ainsi un variant d'un polypeptide parent ayant des propriétés optimisées relatives à l'activité fonctionnelle médiée par la région Fc.

La présente invention se rapporte à une méthode selon la revendication 1, et à un polypeptide comprenant une région Fc mutée selon la revendication 6. Un tel polypeptide est appelé « polypeptide selon l'invention » dans la présente demande.

La description divulgue un polypeptide comprenant une région Fc mutée et présentant une activité fonctionnelle médiée par la région Fc modifiée par rapport à celle d'un polypeptide parent, ladite région Fc comprenant au moins une mutation choisie parmi : G316D, K326E, N315D, N361H, P396L, T350A, V284L, V323I, P352S, A378V, Y436H, V266M, N421T, G385R, K326T, H435R, K447N, N434K, K334N, V397M, E283G, A378T, F423L, A431V, F423S, N325S, P343S, K290E, S375R, F405V, K322E, K340E, N389S, F243I, T307P, N389T, S442F, K248E, Y349H, N286I, T359A, S383R, K334R, T394P, V259A, T393A, P352L, Q418P, V302A, L398P, F423P, S442P, V363I, S383N, S254F, K320E, G402D, I253F, V284A, A431T, N315H, Y319H, C226Y, F405L, T393I, N434S, R255W, A287T, N286Y, A231V, K274R, V308G, K414R, M428T, E345G, F243L, P247T, Q362R, S440N, Y278H, D312G, V262A, V305A, K246R, V308I, E380G, N276S, K439Q, S267G, F423Y, A231T, K320R, L410R, K320M, V412M, T307N, T366A, P230S, Y349S, A339T, K246E, K274E, A231P, I336T, S298N, L234P, S267N, V263A, E333G, V308A, K439R, K392R, S440G, V397I, I336V, Y373D, K288E, L309P, P227S, V379A, K288R, K320T, V282A, I377T, N421S et C261R,
la numérotation étant celle de l'index EU ou équivalent dans Kabat.

Est également décrit un polypeptide comprenant une région Fc mutée et présentant une activité fonctionnelle médiée par la région Fc modifiée par rapport à celle d'un polypeptide parent, ladite région Fc comprenant au moins une combinaison de 2 mutations, ladite combinaison étant choisie parmi :
(i) une mutation choisie parmi 307N, 326E, 326T, 334N, 334R, 352L, 378V, 378T, 394P, 396L, 397M et 421T;
(ii) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N,
la numérotation étant celle de l'index EU ou équivalent dans Kabat et avec la condition que la mutation (i) n'a pas lieu sur le même acide aminé que la mutation (ii).

De préférence, la mutation (i) est choisie parmi 378V, 396L et 397M. De préférence, le polypeptide comprend en outre une mutation choisie parmi 248E, 326T, 333G et 423Y.

De préférence, la mutation (ii) est choisie parmi 226Y, 227S, 230S, 231V, 234P, 243I, 243L, 246R, 246E, 247T, 248E, 253F, 254F, 255W, 259A, 261R, 262A, 263A, 266M, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378T, 379A, 380G, 383R, 385R, 389S, 389T, 392R, 393A, 393I, 394P, 396L, 397I, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N.

Tout au long de la présente demande, la numérotation des résidus dans la région Fc est celle de la chaîne lourde d'immunoglobuline selon l'index EU ou équivalent dans Kabat et al. (Sequences of Proteins of Immunological Interest, 5e éd. Public Health Service, National Institutes of Health, Bethesda, dans le Maryland, 1991). L'expression «index EU ou équivalent dans Kabat» fait référence à la numérotation EU des résidus de l'anticorps humain IgG1, IgG2, IgG3 ou IgG4. Cela est illustré sur le site IMGT (http://www.imgt.org/IMGTScientificChart/Numbering/Hu_IGHGnber.html).

Par «polypeptide» ou «protéine», on entend une séquence comprenant au moins 100 acides aminés attachés de manière covalente.

Par «acide aminé», on entend l'un des 20 acides aminés naturels ou des analogues non naturels.

Le terme « position » signifie une position dans la séquence d'un polypeptide. Pour la région Fc, les positions sont numérotées selon l'indice de l'UE ou équivalent dans Kabat.

Le terme «anticorps» est utilisé dans le sens commun. Il correspond à un tétramère qui comprend au moins une région Fc, et deux régions variables. Les anticorps comprennent notamment les immunoglobulines pleine longueur, les anticorps monoclonaux, les anticorps multi-spécifiques, les anticorps chimériques, les anticorps humanisés et les anticorps entièrement humains. La partie amino-terminale de chaque chaîne lourde comprend une région variable d'environ 100 à 110 acides aminés responsable de la reconnaissance de l'antigène. Dans chaque région variable, trois boucles sont rassemblées pour former un site de liaison à l'antigène. Chacune des boucles est appelée une région déterminant la complémentarité (ci-après dénommé un «CDR»). La partie carboxy-terminale de chaque chaîne lourde définit une région constante principalement responsable de la fonction effectrice.

Les IgG ont plusieurs sous-classes, notamment IgG1, IgG2, IgG3 et IgG4. Les sous-classes d'IgM sont notamment IgM1 et lgM2. Ainsi, par «isotype», on entend l'une des sous-classes d'immunoglobulines définies par les caractéristiques chimiques et antigéniques de leurs régions constantes. Les isotypes connus d'immunoglobulines humaines sont les IgG1, IgG2, IgG3, IgG4, IgA1, lgA2, IgM1, lgM2, IgD et IgE.

Les IgG pleine longueur sont des tétramères et se composent de deux paires identiques de deux chaînes d'immunoglobulines, chaque paire ayant une chaîne légère et une chaîne lourde, chaque chaîne légère comprenant les domaines VL et CL, et chaque chaîne lourde comprenant les domaines VH, Cγ1 (aussi appelé CH1), Cy2 (également appelé CH2), et Cy3 (également appelé CH3). Dans le cadre d'une IgG1 humaine, « CH1 » fait référence aux positions 118 à 215, « CH2 » renvoie aux positions 231 à 340 et « CH3 » se réfère aux positions 341 à 447 selon l'index EU ou équivalent dans Kabat. La chaîne lourde des IgG comprend également un domaine charnière flexible N-terminal qui se réfère aux positions 216 à 230 dans le cas d'IgG1. Le domaine charnière inférieur se réfère aux positions 226 à 230 selon l'index EU ou équivalent dans Kabat. Par "région variable", on entend la région d'une immunoglobuline qui comprend un ou plusieurs domaines Ig sensiblement codés par l'un quelconque des gènes VK, Vλ et/ou VH qui composent les chaînes kappa, lambda, et lourde d'immunoglobuline, respectivement. Les régions variables comprennent les régions déterminant la complémentarité (CDR) et les régions charpente (FR).

Le terme "Fc" ou "région Fc" désigne la région constante d'un anticorps à l'exclusion du premier domaine de région constante d'immunoglobuline (CH1). Ainsi Fc fait référence aux deux derniers domaines (CH2 et CH3) de région constante d'IgG1, et à la charnière flexible N-terminale de ces domaines. Pour une IgG1 humaine, la région Fc correspond au résidu C226 jusqu'à son extrémité carboxy-terminale, soit les résidus de la position 226 à 447, où la numérotation est selon l'index EU ou équivalent dans Kabat. La région Fc utilisée peut comprendre en outre une partie de la région charnière supérieure, située entre les positions 216 à 226 selon l'index EU ou équivalent dans Kabat ; dans ce cas, la région Fc utilisée correspond aux résidus de la position 216 à 447, 217 à 447, 218 à 447, 219 à 447, 220 à 447, 221 à 447, 222 à 447, 223 à 447, 224 à 447 ou 225 à 447, où la numérotation est selon l'index EU ou équivalent dans Kabat. De préférence dans ce cas, la région Fc utilisée correspond aux résidus de la position 216 à 447, où la numérotation est selon l'index EU ou équivalent dans Kabat.

De préférence, la région Fc utilisée est choisie parmi les séquences SEQ ID NO :1 à 10.

Par «polypeptide parent», on entend un polypeptide de référence. Ledit polypeptide parent peut être d'origine naturelle ou synthétique. Dans le contexte de la présente invention, le polypeptide parent comprend une région Fc, appelée « région Fc parente ». Cette région Fc peut être choisie parmi le groupe de régions Fc de type sauvage, leurs fragments et leurs mutants. De préférence, le polypeptide parent comprend une région Fc humaine, de préférence une région Fc d'une IgG1 humaine. Le polypeptide parent peut comprendre des modifications d'acides aminés préexistantes dans la région Fc (par exemple un mutant Fc) par rapport à des régions Fc de type sauvage. Avantageusement, le polypeptide parent est une région Fc isolée (i.e. un fragment Fc en tant que tel), une séquence dérivée d'une région Fc isolée, un anticorps, une protéine de fusion comprenant une région Fc ou un conjugué Fc, cette liste n'étant pas limitative. Par « séquence dérivée d'une région Fc isolée », on entend une séquence comprenant au moins deux régions Fc isolées liées entre elles, telle qu'un scFc (single chain Fc) ou un multimère Fc. Par « protéine de fusion comprenant une région Fc », on entend une séquence polypeptidique fusionnée à une région Fc, ladite séquence polypeptidique étant de préférence choisie parmi les régions variables de tout anticorps, les séquences de liaison d'un récepteur à son ligand, les molécules d'adhésion, les ligands, les enzymes, les cytokines et les chimiokines. Par « conjugué Fc », on entend un composé qui est le résultat du couplage chimique d'une région Fc avec un partenaire de conjugaison. Le partenaire de conjugaison peut être protéique ou non protéique. La réaction de couplage utilise généralement des groupes fonctionnels sur la région Fc et le partenaire de conjugaison. Divers groupes de liaison sont connus dans l'art antérieur comme étant appropriés pour la synthèse d'un conjugué; par exemple, des groupes de liaison homo-ou hétérobifonctionnels sont bien connus (voir, catalogue Pierce Chemical Company, 2005-2006, section technique sur des agents de réticulation, pages 321-350). Parmi les partenaires de conjugaison appropriés, on peut citer les protéines thérapeutiques, les étiquettes, les agents cytotoxiques tels que les agents chimiothérapeutiques, les toxines et leurs fragments actifs. Les toxines appropriées et leurs fragments incluent notamment la toxine de la diphtérie, l'exotoxine A, la ricine, l'abrine, la saporine, la gélonine, la calichéamicine, les auristatines E et F et la mertansine.

Avantageusement, le polypeptide parent - et donc le polypeptide selon l'invention - consiste en une région Fc.

Avantageusement, le polypeptide parent - et donc le polypeptide selon l'invention - est un anticorps.

Enfin, de préférence, le polypeptide parent - et donc le polypeptide selon l'invention - est un polypeptide produit dans le lait d'animaux transgéniques.

Par « mutation », on entend un changement d'au moins un acide aminé de la séquence d'un polypeptide, notamment un changement d'au moins un acide aminé de la région Fc du polypeptide parent. Le polypeptide muté ainsi obtenu est un polypeptide variant ; c'est un polypeptide selon l'invention. Un tel polypeptide comprend une région Fc mutée, par rapport au polypeptide parent. De préférence, la mutation est une substitution, une insertion ou une délétion d'au moins un acide aminé. Par «substitution», on entend le remplacement d'un acide aminé à une position particulière dans une séquence de polypeptide parent par un autre acide aminé. Par exemple, la substitution N434S se réfère à un polypeptide variant, en l'occurrence un variant pour lequel l'asparagine à la position 434 est remplacé par la sérine. Par "insertion d'acide aminé" ou "insertion", on entend l'addition d'un acide aminé à une position particulière dans une séquence de polypeptide parent. Par exemple, l'insertion G>235-236 désigne une insertion de glycine entre les positions 235 et 236. Par « délétion d'acides aminés» ou «délétion», on entend la suppression d'un acide aminé à une position particulière dans une séquence de polypeptide parent. Par exemple, E294del désigne la suppression de l'acide glutamique en position 294. De préférence, le libellé suivant de mutation est utilisé: « 434S » ou « N434S », et signifie que le polypeptide parent comprend l'asparagine en position 434, qui est remplacée par la sérine dans le variant. Dans le cas d'une combinaison de substitutions, le format préféré est le suivant: « 2591/315D/434Y » ou « V259I/N315D/N434Y ». Cela signifie qu'il existe trois substitutions dans le variant, en positions 259, 315 et 434, et que l'acide aminé en position 259 du polypeptide parent, à savoir la valine, est remplacé par l'isoleucine, que l'acide aminé en position 315 du polypeptide parent, soit l'asparagine, est remplacé par l'acide aspartique et que l'acide aminé en position 434 du polypeptide parent, soit l'asparagine, est remplacé par la tyrosine.

Le polypeptide selon l'invention présente une activité fonctionnelle médiée par la région Fc modifiée par rapport à celle du polypeptide parent.

Par "activité fonctionnelle médiée par la région Fc", on entend notamment les fonctions effectrices. L'activité fonctionnelle médiée par la région Fc comprend ainsi notamment la cytotoxicité cellulaire dépendante des anticorps (ADCC ou Antibody-Dependent Cell-mediated Cytotoxicity), la cytotoxicité dépendante du complément (CDC ou Complément Dépendent Cytotoxicity), la phagocytose cellulaire dépendante des anticorps (ADCP), l'activité d'endocytose, la sécrétion de cytokines, ou une combinaison d'au moins deux de ces activités. De préférence, l'activité fonctionnelle médiée par la région Fc considérée dans l'invention est choisie parmi l'ADCC, la CDC et leur combinaison. Cette activité fonctionnelle peut être évaluée par des procédés bien connus de l'art antérieur, tels que ceux décrits dans les exemples (voir notamment les points 4.4 et 4.5 des exemples).

L'activité fonctionnelle médiée par la région Fc du polypeptide selon l'invention est augmentée par rapport à celle du polypeptide parent.

Le polypeptide selon l'invention présente une activité fonctionnelle médiée par la région Fc augmentée par rapport à celle du polypeptide parent. De préférence, le polypeptide selon l'invention présente une activité fonctionnelle médiée par la région Fc augmentée, par rapport à celle du polypeptide parent, d'un ratio au moins égal à 2, de préférence supérieur à 5, de préférence supérieur à 10, de préférence supérieur à 15, de préférence supérieur à 20, de préférence supérieur à 25, et de préférence supérieur à 30.

De préférence, la région Fc mutée du polypeptide selon l'invention a une affinité modifiée pour au moins un des récepteurs de la région Fc (FcR) choisi parmi le complément C1q, FcgRIIIa (CD16a), FcgRIIa (CD32a), et FcgRIIb (CD32b). Le complément C1q est en effet impliqué dans l'activité CDC. Le récepteur FcgRIIIa (CD16a) est, quant à lui, impliqué dans l'ADCC ; il présente un polymorphisme V/F en position 158. Le récepteur FcgRIIa (CD32a) est, quant à lui, impliqué dans l'activation plaquettaire et la phagocytose; il présente un polymorphisme H/R en position 131. Enfin, le récepteur FcgRIIb (CD32b) est impliqué dans l'inhibition de l'activité cellulaire.

Préférentiellement, ladite région Fc mutée a une affinité augmentée pour au moins l'un des FcR. Préférentiellement, l'affinité est augmentée, par rapport à celle du Fc parent, d'un ratio au moins égal à 2, de préférence supérieur à 5, de préférence supérieur à 10, de préférence supérieur à 15, de préférence supérieur à 20, de préférence supérieur à 25, et de préférence supérieur à 30. En d'autres termes, l'affinité de la région Fc mutée pour un FcR est supérieure à celle du polypeptide parent. Alternativement, ladite région Fc mutée a une affinité diminuée pour au moins l'un des FcR. Préférentiellement, l'affinité est diminuée, par rapport à celle du Fc parent, d'un ratio au moins égal à 2, de préférence supérieur à 5, de préférence supérieur à 10, de préférence supérieur à 15, de préférence supérieur à 20, de préférence supérieur à 25, et de préférence supérieur à 30. En d'autres termes, l'affinité de la région Fc mutée pour un FcR est inférieure à celle du polypeptide parent.

L'affinité d'un polypeptide comprenant une région Fc pour un FcR peut être évaluée par des procédés bien connus de l'art antérieur. Par exemple, l'homme de l'art peut déterminer l'affinité (Kd) en utilisant la résonance plasmonique de surface (SPR). Alternativement, l'homme de l'art peut effectuer un test ELISA approprié. Un dosage ELISA approprié permet de comparer les forces de liaison du Fc parent et du Fc muté. Les signaux détectés spécifiques du Fc muté et du Fc parent sont comparés. L'affinité de liaison peut être indifféremment déterminée en évaluant les polypeptides entiers ou en évaluant les régions Fc isolées de ceux-ci.

Est également divulguée une région Fc mutée du polypeptide comprenant de 1 à 20 mutations par rapport au polypeptide parent, de préférence de 2 à 20 mutations. Par «de 1 à 20 modifications d'acides aminés», on englobe 1, 2, 3, 4, 5, 6, 7, 8, 9, 10, 11, 12, 13, 14, 15, 16, 17, 18, 19 et 20 mutations d'acides aminés. De préférence, elle comprend de 1 à 15 mutations, de préférence de 2 à 15 mutations, de préférence de 1 à 10 mutations par rapport au polypeptide parent, de préférence de 2 à 10 mutations.

Est également divulguée une région Fc mutée du polypeptide comprenant au moins une combinaison de 2 mutations, ladite combinaison étant choisie parmi :
(i) une mutation choisie parmi 378V, 396L et 397M;
(ii) au moins une mutation choisie parmi 231V, 248E, 286I, 286Y, 290E, 298N, 308A, 315D, 316D, 326E, 333G, 334N, 334R, 336T, 352S, 361H, 366A, 378T, 396L, 397M, 412M, 421T, 423Y et 447N,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide comprenant au moins une combinaison de 2 mutations, ladite combinaison étant choisie parmi :
(i) une mutation choisie parmi 378V, 396L et 397M;
(ii) au moins une mutation choisie parmi 248E, 316D, 326E, 333G, 378T, 396L et 421T,
la numérotation étant celle de l'index EU ou équivalent dans Kabat et avec la condition que la mutation (i) n'a pas lieu sur le même acide aminé que la mutation (ii).

Est également divulgué un polypeptide comprenant une région Fc mutée présentant des activités fonctionnelles ADCC et CDC augmentées par rapport à celle d'un polypeptide parent, caractérisé en ce que ladite région Fc comprend au moins une combinaison de 2 mutations, ladite combinaison étant choisie parmi :
(i) une mutation choisie parmi 378V, 396L et 397M;
(ii) au moins une mutation choisie parmi 248E, 316D, 326E, 333G, 378T, 396L et 421T,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide comprenant au moins une combinaison de 2 mutations, ladite combinaison étant choisie parmi :
(i) la mutation 378V;
(ii) au moins une mutation choisie parmi 298N et 336T,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide comprenant au moins une combinaison de 2 mutations, ladite combinaison étant choisie parmi :
(i) une mutation choisie parmi 378V, 396L et 397M;
(ii) au moins une mutation choisie parmi 231V, 286I, 286Y, 290E, 315D, 334N, 352S, 361H, 366A, 378T, 397M, 412M, 421T et 423Y,
la numérotation étant celle de l'index EU ou équivalent dans Kabat et avec la condition que la mutation (i) n'a pas lieu sur le même acide aminé que la mutation (ii).

Est également divulgué un polypeptide comprenant une région Fc mutée présentant une activité fonctionnelle ADCC augmentée par rapport à celle d'un polypeptide parent, caractérisé en ce que ladite région Fc comprend au moins une combinaison de 2 mutations, ladite combinaison étant choisie parmi :
(i) une mutation choisie parmi 378V, 396L et 397M;
(ii) au moins une mutation choisie parmi 231V, 286I, 286Y, 298N, 290E, 315D, 334N, 336T, 352S, 361H, 366A, 378T, 397M, 412M, 421T et 423Y,
la numérotation étant celle de l'index EU ou équivalent dans Kabat et avec la condition que la mutation (i) n'a pas lieu sur le même acide aminé que la mutation (ii).

De préférence, la région Fc mutée du polypeptide selon l'invention comprend au moins une combinaison de 2 mutations, ladite combinaison étant choisie parmi :
(i) la mutation 378V;
(ii) au moins une mutation choisie parmi 248E, 308A, 334R, 447N,
la numérotation étant celle de l'index EU ou équivalent dans Kabat et avec la condition que la mutation (i) n'a pas lieu sur le même acide aminé que la mutation (ii).

De préférence, le polypeptide selon l'invention comprend une région Fc mutée présentant une activité fonctionnelle CDC augmentée par rapport à celle d'un polypeptide parent, caractérisé en ce que ladite région Fc comprend au moins une combinaison de 2 mutations, ladite combinaison étant choisie parmi :
(i) la mutation 378V;
(ii) au moins une mutation choisie parmi 248E, 308A, 334R, 447N,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide comprenant une combinaison de mutations choisie parmi les combinaisons :
K320E/T394P/G402D
K290E/K320E/T350A/P396L
T359A/S383R/V397M

Est également divulguée une région Fc mutée du polypeptide ayant une affinité améliorée pour le complément C1q, et comprenant au moins une combinaison de 2 mutations, ladite combinaison comprenant :
i) une mutation choisie parmi 378V, 378T, 396L, 421T, 334R et 326E ; et
ii) au moins une mutation choisie parmi 361H, 290E, 316D, 248E, 410R, 421T, 334R, 394P, 307P, 447N, 378V, 284L, 421T, 396L, 286I, 315D et 397M,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide ayant une affinité améliorée pour le récepteur FcgRIIIa (CD16a), et comprenant au moins une combinaison de 2 mutations, ladite combinaison comprenant:
i) une mutation choisie parmi 378V, 326E, 397M, 334N et 396L ; et
ii) au moins une mutation choisie parmi 316D, 397M, 334N, 248E, 231V, 246R, 336T, 421T, 361H, 366A, 439R, 290E, 394P, 307P, 378V, 378T, 286I, 286Y et 298N,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide ayant une affinité augmentée pour le récepteur FcgRIIa (CD32a), et comprenant au moins une combinaison de 2 mutations, ladite combinaison comprenant:
i) une mutation choisie parmi 378V, 326E, 397M, 307N, 394P, 326T, 396L et 334N ; et
ii) au moins une mutation choisie parmi : 316D, 334R, 334N, 323I, 231V, 246R, 336T, 378T, 286Y, 286I, 352S, 383R, 359A, 421T, 361H, 315D, 366A, 290E, 307P et 439R,
la numérotation étant celle de l'index EU ou équivalent dans Kabat et avec la condition que la mutation (i) n'a pas lieu sur le même acide aminé que la mutation (ii).

De préférence, la mutation (ii) est choisie parmi : 316D, 334R, 334N, 323I, 231V, 246R, 336T, 378T, 286Y, 286I, 352S, 383R, 359A, 421T, 361H, 366A, 290E, 307P et 439R.

Est également divulguée une région Fc mutée du polypeptide ayant une affinité augmentée pour le récepteur FcgRIIb (CD32b), et comprenant au moins une combinaison de 2 mutations, ladite combinaison comprenant :
i) une mutation choisie parmi 326E, 326T, 378V, 397M, 352L, 394P, 396L et 421T ; et
ii) au moins une mutation choisie parmi 316D, 334R, 248E, 334N, 418P, 231V, 320E, 402D, 359A, 383R, 421T et 361H,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide ayant une activité CDC augmentée, et comprenant au moins une combinaison de 2 mutations, ladite combinaison comprenant :
i) une mutation choisie parmi 378V, 378T, 396L, 421T, 334R et 326E ; et
ii) au moins une mutation choisie parmi 361H, 290E, 316D, 248E, 410R, 421T, 334R, 394P, 307P, 447N, 378V, 284L, 421T, 396L, 286I, 315D et 397M,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide ayant une activité ADCC augmentée, et comprenant au moins une combinaison de 2 mutations, ladite combinaison comprenant :
i) une mutation choisie parmi 378V, 326E, 397M, 334N et 396L ; et
ii) au moins une mutation choisie parmi 316D, 397M, 334N, 248E, 231V, 246R, 336T, 421T, 361H, 366A, 439R, 290E, 394P, 307P, 378V, 378T, 286I, 286Y et 298N,
la numérotation étant celle de l'index EU ou équivalent dans Kabat et avec la condition que la mutation (i) n'a pas lieu sur le même acide aminé que la mutation (ii).

Est également divulguée une région Fc mutée du polypeptide comprenant au moins une combinaison de 3 mutations, ladite combinaison comprenant :
(i) une mutation choisie parmi 326E, 326T, 352L, 378V, 378T, 396L, 397M, 421T, 334N, 334R, 307N et 394P ; et
(ii) au moins 2 mutations choisies parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P et 447N,
la numérotation étant celle de l'index EU ou équivalent dans Kabat et avec la condition que la mutation (i) n'a pas lieu sur le même acide aminé que la mutation (ii).

De préférence, les au moins 2 mutations (ii) sont choisies parmi 226Y, 227S, 230S, 231V, 234P, 243I, 243L, 246R, 246E, 247T, 248E, 253F, 254F, 255W, 259A, 261R, 262A, 263A, 266M, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378T, 379A, 380G, 383R, 385R, 389S, 389T, 392R, 393A, 393I, 394P, 396L, 397I, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N.

Plus préférentiellement, la région Fc mutée du polypeptide comprend au moins une combinaison de 4 mutations, ladite combinaison comprenant au moins une mutation (i) telle que décrite ci-dessus, et au moins 3 mutations (ii) telles que décrites ci-dessus, la numérotation étant celle de l'index EU ou équivalent dans Kabat et avec la condition que la mutation (i) n'a pas lieu sur le même acide aminé que la mutation (ii).

Plus préférentiellement, la région Fc mutée du polypeptide comprend au moins une combinaison de 5 mutations, ladite combinaison comprenant au moins une mutation (i) telle que décrite ci-dessus, et au moins 4 mutations (ii) telles que décrites ci-dessus, la numérotation étant celle de l'index EU ou équivalent dans Kabat et avec la condition que la mutation (i) n'a pas lieu sur le même acide aminé que la mutation (ii).

La présente invention a également pour objet une composition de polypeptides selon l'invention, lesdits polypeptides purifiés possédant sur leur site de glycosylation Asn297 des N-glycannes dont le taux de fucosylation est inférieur à 65%, de préférence inférieur à 50%, de préférence encore inférieur à 40%. De préférence, lesdits polypeptides purifiés possèdent sur leur site de glycosylation Asn297 une structure glycannique de type biantenné, avec des chaînes courtes, une faible sialylation, présentant des mannoses terminaux et/ou des N-acétylglucosamines terminaux non intercalaires.

Plus préférentiellement, lesdits polypeptides purifiés présentent une teneur supérieure à 60% pour les formes G0+G1+G0F+G1F, les formes G0F+G1F étant inférieures à 50%. Plus préférentiellement, lesdits polypeptides purifiés présentent une teneur supérieure à 60% pour les formes G0+G1+G0F+G1F, la teneur en fucose étant inférieure à 65%. Plus préférentiellement, lesdits polypeptides purifiés présentent une teneur inférieure à 40% pour les formes G1F+G0F.

La présente invention a également pour objet une composition pharmaceutique comprenant (i) un polypeptide selon l'invention ou une composition telle que décrite au paragraphe précédent, et (ii) au moins un excipient pharmaceutiquement acceptable.

La présente invention a également pour objet le polypeptide selon l'invention ou la composition telle que décrite précédemment, pour son utilisation comme médicament.

Comme indiqué précédemment, avantageusement, le polypeptide parent - et donc le polypeptide selon l'invention - est un anticorps. Dans ce cas, l'anticorps peut être dirigé contre un antigène choisi parmi un antigène tumoral, un antigène viral, un antigène bactérien, un antigène fongique, une toxine, une cytokine membranaire ou circulante et un récepteur membranaire.

Lorsque l'anticorps est dirigé contre un antigène tumoral, son utilisation convient particulièrement dans le traitement de cancers. Par « cancer », on entend toute condition physiologique caractérisée par une prolifération anormale des cellules. Des exemples de cancers incluent notamment les carcinomes, les lymphomes, les blastomes, les sarcomes (incluant les liposarcomes), les tumeurs neuroendocrines, les mésothéliomes, les méningiomes, les adénocarcinomes, les mélanomes, les leucémies et les pathologies lymphoïdes malignes, cette liste n'étant pas exhaustive.

Lorsque l'anticorps est dirigé contre un antigène viral, son utilisation convient particulièrement dans le traitement des infections virales. Les infections virales sont notamment des infections dues au VIH, à un rétrovirus, à un virus Coxsackie, au virus de la variole, de la grippe, de la fièvre jaune, du Nil occidental, à un cytomégalovirus, à un rotavirus ou au virus de l'hépatite B ou C, cette liste n'étant pas exhaustive. Lorsque l'anticorps est dirigé contre une toxine, son utilisation convient particulièrement dans le traitement des infections bactériennes, par exemple les infections par la toxine tétanique, la toxine diphtérique, les toxines du charbon *Bacillus anthracis,* ou encore dans le traitement des infections par les toxines botuliques, les toxines de la ricine, les shigatoxines, cette liste n'étant pas exhaustive.

Lorsque l'anticorps est dirigé contre une cytokine, son utilisation convient particulièrement dans le traitement des maladies inflammatoires et/ou auto-immunes. Les maladies inflammatoires et/ou auto-immunes incluent notamment le purpura thrombotique thrombocytopénique (PTI), le rejet de greffes ou d'organes, la maladie du greffon contre l'hôte, la polyarthrite rhumatoïde, le lupus érythémateux disséminé, les différents types de sclérose, le syndrome de Sjögren primitif (ou syndrome de Gougerot-Sjögren), les polyneuropathies auto-immunes comme la sclérose en plaques, le diabète de type I, les hépatites auto-immunes, la spondylarthrite ankylosante, le syndrome de Reiter, l'arthrite de goutte, la maladie coeliaque, la maladie de Crohn, la thyroïdite chronique d'Hashimoto (hypothyroïdie), la maladie d'Adisson, les hépatites auto-immunes, la maladie de Basedow (hyperthyroïdie), la colite ulcérative, les vascularites comme les vascularites systémiques associées aux ANCA (anticorps anti-cytoplasme des neutrophiles), les cytopénies auto-immunes et autres complications hématologiques de l'adulte et de l'enfant, telles que les thrombopénies auto-immunes aiguës ou chroniques, les anémies hémolytiques auto-immunes, la maladie hémolytique du nouveau-né (MHN), la maladie des agglutinines froides, l'hémophilie acquise auto-immune ; le syndrome de Goodpasture, les néphropathies extra-membraneuses, les affections cutanées bulleuses auto-immunes, la myasthénie réfractaire, les cryoglobulinémies mixtes, le psoriasis, l'arthrite chronique juvénile, les myosites inflammatoires, les dermatomyosites et les affections systémiques auto-immunes de l'enfant incluant le syndrome des antiphospholipides, la maladie des tissus conjonctifs, l'inflammation auto-immune pulmonaire, le syndrome Guillain-Barré, la polyradiculonévrite inflammatoire demyélisante chronique (PDCI), la thyroïdite auto-immune, le mellitis, le myasthenia gravis, la maladie autoimmune inflammatoire de l'oeil, la neuromyélite optique (maladie de Devic), les sclérodermies, le pemphigus, le diabète par insulinorésistance, la polymyosite, l'anémie de Biermer, la glomérulonéphrite, la maladie de Wegener, la maladie de Horton, la périarthrite noueuse et le syndrome de Churg et Strauss, la maladie de Still, la polychondrite atrophiante, la maladie de Behçet, la gammapathie monoclonale, la granulomatose de Wegener, le lupus, la rectocolite hémorragique, le rhumatisme psoriasique, la sarcoïdose, la colite collagène, la dermatite herpétiforme, la fièvre méditerranéenne familiale, la glomérulonéphrite à dépôts d'IgA, le syndrome myasthénique de Lambert-Eaton, l'ophtalmie sympathique, le syndrome de Fiessinger-Leroy-Reiter et le syndrome uvéo-méningo-encéphalique.

D'autres maladies inflammatoires sont également comprises, comme par exemple le syndrome de détresse respiratoire aiguë (ARDS), l'arthrite septique aiguë, l'arthrite adjuvante, l'encéphalomyélite allergique, la rhinite allergique, la vasculite allergique, l'allergie, l'asthme, l'athérosclérose, l'inflammation chronique due à des infections bactériennes ou virales chroniques, la bronchopneumopathie chronique obstructive (MPOC), les maladies coronariennes, l'encéphalite, les maladies inflammatoires de l'intestin, l'ostéolyse inflammatoire, l'inflammation associée à des réactions d'hypersensibilité aiguë et retardée, l'inflammation associée à des tumeurs, une lésion nerveuse périphérique ou des maladies démyélinisantes, une inflammation associée à un traumatisme des tissus tels que les brûlures et l'ischémie, l'inflammation due à une méningite, le syndrome de défaillance multiviscérale (multiple organ dysfunction syndrome, MODS), la fibrose pulmonaire, la septicémie et le choc septique, le syndrome de Stevens-Johnson, l'arthrite indifférenciée, et les spondylarthropathies indifférenciées.

Dans un mode de réalisation particulier de l'invention, la maladie auto-immune est le purpura thrombotique idiopatique (PTI) et le la polyradiculonévrite inflammatoire demyélisante chronique (PDCI).

Est également divulguée une méthode de production d'un polypeptide comprenant une région Fc et présentant une activité fonctionnelle médiée par la région Fc modifiée par rapport à celle d'un polypeptide parent, ladite méthode comprenant une étape d'introduction d'au moins une mutation dans ladite région Fc choisie parmi :
G316D, K326E, N315D, N361H, P396L, T350A, V284L, V323I, P352S, A378V, Y436H, V266M, N421T, G385R, K326T, H435R, K447N, N434K, K334N, V397M, E283G, A378T, F423L, A431V, F423S, N325S, P343S, K290E, S375R, F405V, K322E, K340E, N389S, F243I, T307P, N389T, S442F, K248E, Y349H, N286I, T359A, S383R, K334R, T394P, V259A, T393A, P352L, Q418P, V302A, L398P, F423P, S442P, V363I, S383N, S254F, K320E, G402D, I253F, V284A, A431T, N315H, Y319H, C226Y, F405L, T393I, N434S, R255W, A287T, N286Y, A231V, K274R, V308G, K414R, M428T, E345G, F243L, P247T, Q362R, S440N, Y278H, D312G, V262A, V305A, K246R, V308I, E380G, N276S, K439Q, S267G, F423Y, A231T, K320R, L410R, K320M, V412M, T307N, T366A, P230S, Y349S, A339T, K246E, K274E, A231P, I336T, S298N, L234P, S267N, V263A, E333G, V308A, K439R, K392R, S440G, V397I, I336V, Y373D, K288E, L309P, P227S, V379A, K288R, K320T, V282A, I377T, N421S et C261R,
la numérotation étant celle de l'index EU ou équivalent dans Kabat.

Est également divulguée une méthode de production d'un polypeptide comprenant une région Fc et présentant une activité fonctionnelle médiée par la région Fc modifiée par rapport à celle d'un polypeptide parent, ladite méthode comprenant une étape d'introduction d'au moins une combinaison de 2 mutations, ladite combinaison étant choisie parmi :
(i) une mutation choisie parmi 326E, 326T, 352L, 378V, 378T, 396L, 397M, 421T, 334N, 334R, 307N et 394P ; et
(ii) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N,
la numérotation étant celle de l'index EU ou équivalent dans Kabat et avec la condition que la mutation (i) n'a pas lieu sur le même acide aminé que la mutation (ii).

De préférence, la mutation (ii) est choisie parmi 226Y, 227S, 230S, 231V, 234P, 243I, 243L, 246R, 246E, 247T, 248E, 253F, 254F, 255W, 259A, 261R, 262A, 263A, 266M, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378T, 379A, 380G, 383R, 385R, 389S, 389T, 392R, 393A, 393I, 394P, 396L, 397I, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N.

Est également divulguée une méthode d'augmentation de la liaison d'un polypeptide comprenant une région Fc à au moins un des récepteurs de la région Fc (FcR) choisi parmi le récepteur C1q, FcgRIIIa (CD16a), et FcgRIIa (CD32a), et le FcgRIIb (CD32b), ladite méthode comprenant une étape d'introduction d'au moins une mutation dans ladite région Fc choisie parmi :
G316D, K326E, N315D, N361H, P396L, T350A, V284L, V323I, P352S, A378V, Y436H, V266M, N421T, G385R, K326T, H435R, K447N, N434K, K334N, V397M, E283G, A378T, F423L, A431V, F423S, N325S, P343S, K290E, S375R, F405V, K322E, K340E, N389S, F243I, T307P, N389T, S442F, K248E, Y349H, N286I, T359A, S383R, K334R, T394P, V259A, T393A, P352L, Q418P, V302A, L398P, F423P, S442P, V363I, S383N, S254F, K320E, G402D, I253F, V284A, A431T, N315H, Y319H, C226Y, F405L, T393I, N434S, R255W, A287T, N286Y, A231V, K274R, V308G, K414R, M428T, E345G, F243L, P247T, Q362R, S440N, Y278H, D312G, V262A, V305A, K246R, V308I, E380G, N276S, K439Q, S267G, F423Y, A231T, K320R, L410R, K320M, V412M, T307N, T366A, P230S, Y349S, A339T, K246E, K274E, A231P, I336T, S298N, L234P, S267N, V263A, E333G, V308A, K439R, K392R, S440G, V397I, I336V, Y373D, K288E, L309P, P227S, V379A, K288R, K320T, V282A, I377T, N421S et C261R,
la numérotation étant celle de l'index EU ou équivalent dans Kabat.

Est également divulguée une méthode d'augmentation de la liaison d'un polypeptide comprenant une région Fc à au moins un des récepteurs de la région Fc (FcR) choisi parmi le récepteur C1q, FcgRIIIa (CD16a), et FcgRIIa (CD32a), et le FcgRIIb (CD32b), ladite méthode comprenant une étape d'introduction d'au moins une combinaison de 2 mutations, ladite combinaison étant choisie parmi :
(i) une mutation choisie parmi 326E, 326T, 352L, 378V, 378T, 396L, 397M, 421T, 334N, 334R, 307N et 394P ; et
(ii) au moins une mutation choisie parmi 226Y, 227S, 230S, 231V, 234P, 243I, 243L, 246R, 246E, 247T, 248E, 253F, 254F, 255W, 259A, 261R, 262A, 263A, 266M, 267N, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 315D, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378V, 378T, 379A, 380G, 383R, 385R, 389S, 389T, 392R, 393A, 393I, 394P, 396L, 397I, 397M, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N,
la numérotation étant celle de l'index EU ou équivalent dans Kabat et avec la condition que la mutation (i) n'a pas lieu sur le même acide aminé que la mutation (ii).

De préférence, la mutation (ii) est choisie parmi 226Y, 227S, 230S, 231V, 234P, 243I, 243L, 246R, 246E, 247T, 248E, 253F, 254F, 255W, 259A, 261R, 262A, 263A, 266M, 267G, 274E, 274R, 276S, 278H, 282A, 283G, 284L, 286I, 286Y, 287T, 288E, 288R, 290E, 298N, 302A, 305A, 307P, 308A, 308I, 308G, 309P, 312G, 316D, 319H, 320T, 320R, 320M, 322E, 323I, 325S, 333G, 334N, 334R, 336T, 339T, 340E, 343S, 345G, 349S, 349H, 350A 352S, 359A, 361H, 362R, 363I, 366A, 373D, 375R, 377T, 378T, 379A, 380G, 383R, 385R, 389S, 389T, 392R, 393A, 393I, 394P, 396L, 397I, 398P, 405V, 405L, 410R, 412M, 414R, 421T, 421S, 423L, 423Y, 423S, 423P, 428T, 431V, 431T, 434K, 434S, 435R, 436H, 439R, 440G, 440N, 442F, 442P, et 447N.

Les séquences décrites dans la présente demande peuvent être résumées comme suit:

| **SEQ ID NO :** | **Protéine** |
|---|---|
| 1 | Région Fc d'IgG1 humaine G1m1,17 (résidus 226-447 selon l'index EU ou équivalent dans Kabat) sans région charnière supérieure N-terminale |
| 2 | Région Fc d'IgG2 humaine sans région charnière supérieure N-terminale |
| 3 | Région Fc d'IgG3 humaine sans région charnière supérieure N-terminale |
| 4 | Région Fc d'IgG4 humaine sans région charnière supérieure N-terminale |
| 5 | Région Fc d'IgG1 humaine G1m3 sans région charnière supérieure N-terminale |
| 6 | Région Fc d'IgG1 humaine G1m1,17 avec région charnière supérieure N-terminale (résidus 216-447 selon l'index EU ou équivalent dans Kabat) |
| 7 | Région Fc d'IgG2 humaine avec région charnière supérieure N-terminale |
| 8 | Région Fc d'IgG3 humaine avec région charnière supérieure N-terminale |
| 9 | Région Fc d'IgG4 humaine avec région charnière supérieure N-terminale |
| 10 | Région Fc d'IgG1 humaine G1m3 avec région charnière supérieure N-terminale |
| 11 et 12 | Amorces MG_619 et MG_621 |

La présente invention sera mieux comprise à la lecture des exemples suivants.

### EXEMPLES

### EXEMPLE 1: Identification de polypeptides à région Fc mutée selon l'invention et caractérisation desdits polypeptides

### I. Matériels et méthodes

### 1. Constructions des banques de la région Fc humaine

Le gène Fc humain codant les acides aminés 226-447 (index EU ou équivalent dans Kabat), i.e. un polypeptide comprenant une région Fc, dérivé d'une chaîne lourde d'IgG1 humaine et ayant l'allotype G1m1.17 (SEQ ID NO : 1), (Poul MA et al, Eur J. Immunol 25 (7): 2005-2009, 1995) a été cloné dans le vecteur phagemide pMG58 (pMG58_Fc226) en tant que fragment BamHI / EcoRI en utilisant des protocoles de PCR standard. Plusieurs banques complètement randomisées ont été générées en utilisant le procédé MUTAGEN^{™} (WO02/038756) qui utilise des ADN-polymérases humaines (mutases) à faible fidélité pour introduire des mutations aléatoires de manière homogène sur la séquence cible entière. Trois mutases distinctes (pol β, pol η et pol 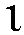 ) ont été utilisées dans des conditions différentes pour créer des profils de mutations complémentaires. Ces polymérases humaines ont été produites et purifiées comme décrit précédemment (Mondon et al. J. Biotechnol 2 : 76-82 (2007), Emond et al Protein Eng Des SeI 21:267-274, (2008)).

### 1.1. Mutagenèse avec le procédé MUTAGEN^{™}

Le procédé MUTAGEN^{™} a été décrit dans la demande WO02/038756.

Brièvement, le gène Fc humain (gène Fc) a été répliqué à l'aide de mutases en utilisant l'amorce 5' MG_619 : 5'-AGTACTGACTCTACCTAGGATCCTGCCCACCGTGC-3' (SEQ ID NO:11) et l'amorce 3' MG_621 : 5'-ACTGCTCGATGTCCGTACTATGCGGCCGCGAATTC-3' (SEQ ID NO :12). Un mélange contenant 0.6µg du plasmide pMG58_Fc226 comme modèle (région Fc sauvage ou variants sélectionnés), les amorces MG_619 et MG_621 (250nM chacune) et le tampon de réplication approprié (voir les détails ci-dessous) a été traité pendant 5 min à 95°C et immédiatement refroidi à 4°C pour dénaturer les brins d'ADN. Pour pol β, le tampon de réplication était 50mM Tris HCl pH 8,8, 10mm MgCl2, 10mM KCI, 1 mM DTT et 1% (v/v) de glycérol. Le tampon de réplication pour la pol η (ou pol η et pol ι) était 25 mM Tris HCl pH 7,2, 5 mM MgCl2, 10mm KCl, 1 mM DTT et 2,5% (v/v) de glycérol. Après l'étape de dénaturation, les réplications mutagènes ont été réalisées en ajoutant 50 µM de dATP / dCTP, 100 µM de dTTP / dGTP et 1 µg de pol β ou 100 µM de dNTP et 1 µg de pol η (ou pol η et pol 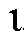 , 1 µg de chaque mutase). La réaction de réplication a été effectuée à 37°C pendant deux heures. Les produits de réplication ont été ensuite concentrés et dessalés sur colonnes Microcon (Millipore).

### 1.2. Amplification sélective et clonage des fragments mutés

Les produits de réplication précédemment obtenus ont été amplifiés par une PCR sélective avec des amorces de queue. Les amorces (MG_619 MG_621) ont été conçues avec une queue qui est non-complémentaire du modèle permettant l'amplification spécifique des fragments d'ADN synthétisés par les mutases. Une fraction des produits de réplication a été ajoutée à un mélange contenant le tampon de PCR (20mM Tris-HCl pH 8,4, 50mM KCl), 1,5mM MgCl2, 10pmol des amorces 5' et 3', 200pM de dNTPs et 1,25 U d'ADN Taq polymerase Platinum (Invitrogen). Les cycles de PCR étaient les suivants, premier cycle: 2 min. à 94°C, 10 sec. à 64°C, 30 sec. à 75°C, 1 min. à 94°C, puis 30 cycles sélectifs: 20 sec. à 94°C et 30 sec. à 75°C.

Les produits de réplication amplifiés ont été purifiés sur 1% (p / v) de gels d'agarose, digérés avec BamHI et EcoRI et clonés dans le vecteur pMG58. Les mélanges de ligature ont été transformés dans des cellules électrocompétentes E. coli XL1-Blue et ensuite étalés sur un milieu solide 2YT (16 g/l de peptone, 10 g/l d'extrait de levure, 5 g/l de NaCl, 15 g/l d'agar) additionné de 100 µg/ml d'ampicilline et 1% (p/v) de glucose. Après la croissance, le nombre de colonies a été déterminé pour estimer la taille des banques et au moins 48 clones par banque ont été soumis à une PCR et à un séquençage ADN à haut débit. Les cellules ont été resuspendues dans le milieu 2YT avec 15% de glycérol, congelées et conservées à -80°C.

### 1.3. Construction de la banque Mut3

Une première banque a été obtenue en utilisant pol β sur le gène Fc sauvage et contenait 3,2×10⁶ clones (appelée Mut1.1). L'ADN de cette première banque a été utilisé pour générer les deuxième et troisième banques, en utilisant respectivement pol β (3,8×10⁶ clones, Mut1.2) et pol η et i (3,0×10⁶ clones, Mut1.3). Cette stratégie en deux étapes cumulatives de réplication a permis d'augmenter le taux de mutation. La quatrième banque a été réalisée avec pol η seule sur le gène Fc sauvage (1,0×10⁶ clones, Mut1.4). Enfin, ces quatre banques ont été proportionnellement mélangées pour obtenir la banque finale appelée Mut1, représentant 1,1×10⁷ clones différents.

Deux banques différentes ont ensuite été construites en utilisant un pool d'ADN de 42 mutants simples et doubles isolés sur le récepteur FcRn. Une première banque a été obtenue en utilisant pol β (1,9×10⁷ clones, Mut2.1) et une seconde banque avec pol η (1×10⁶ clones, Mut2.2). Enfin, ces deux banques ont été proportionnellement mélangées pour obtenir la banque finale appelée Mut2, soit 2×10⁷ clones différents.

Une nouvelle banque a été obtenue en utilisant pol β sur le gène Fc sauvage, avec une diversité de 4,7×10⁷ clones.

Enfin, les banques Mut1, Mut2 et la nouvelle banque ont été proportionnellement mélangées pour obtenir la banque finale appelée Mut3, soit 7,8×10⁷ clones différents. Finalement, la banque Mut3 contient 3×10⁷ clones mutés en phase avec en moyenne 2,2 acides aminés mutés par Fc.

### 1.4. Construction de la banque Mut4sel

Une banque de variants Fc a été construite à partir de 36 clones de liaison améliorée pour le C1q et/ou le CD16aV sélectionnés à partir de la banque Mut3 (1,6 acides aminés mutés par Fc en moyenne). La banque Mut4 a été obtenue en utilisant un mélange équimolaire des polymérases pol β et pol η (dans le tampon de réplication de la pol β). La banque Mut4 a une diversité de 1,3×10⁷ clones, avec 77% de clones en phase et en moyenne 2,6 acides aminés mutés par Fc.

Cette banque a été clonée dans le vecteur pMG93, vecteur de sélection développé pour le Fc, qui permet la sélection des séquences codantes (ORFs) sur milieu ampicilline grâce à une fusion avec le gène de la □-lactamase. Cette construction a été transformée dans les bactéries non suppressives HB2151, ce qui permet d'éliminer les codons TAGs qui sont reconnus comme des codons stop dans cette souche. Les clones de cette banque ont été étalés sur des boîtes de milieu gélosé contenant de faibles concentrations d'ampicilline, à une faible densité, de manière à réaliser la sélection des ORFs, les clones viables étant ensuite récupérés et congelés. L'étalement sur 500 boîtes de Pétri (120x120) a permis de recouvrir la totalité de la banque Mut4. La banque ainsi sélectionnée a été notée Mut4sel et contient 92% de clones en phase, soit 1×10⁷ clones mutés en phases avec en moyenne 2,5 acides aminés mutés par Fc. La banque Mut4sel a ensuite été sous-clonée dans le phagemide pMG58, dans les bactéries XL1-Blue, afin de permettre la sélection par « phage display » sur les cibles.

### 1.5. Construction de la banque Mut5

Une banque de variants Fc a été construite à partir de 42 clones de liaison améliorée pour le C1q et/ou l'un des récepteurs FcgRs sélectionnés à partir de la banque Mut4sel (2,4 acides aminés mutés par Fc en moyenne). La banque Mut5 a été obtenue en utilisant la polymérase pol β seule de manière à obtenir un faible taux de mutation. Finalement, la banque Mut5 obtenue contient 1,2×10⁷ clones avec 95% de clones en phase avec 3,1 acides aminés mutés par Fc en moyenne. La bonne qualité de cette banque n'a pas nécessité la sélection des ORFs comme réalisé pour la banque Mut4.

### 2. Expression en phage display des banques de Fc et sélection des variants améliorés

Au cours des étapes de sélection, les banques Mut3, Mut4sel et Mut5 ont été exprimées à la surface du bactériophage M13 en utilisant des procédures standards (Smith GP, Science 228: 1315 (1985)). Des bactéries E. coli XL1-Blue, contenant la banque à exprimer clonée dans le vecteur pMG58, ont été cultivées dans 60 ml de milieu 2YT additionné de 100 µg/ml d'ampicilline, 15 µg/ml de tétracycline et 1% (p/v) de glucose à 30°C. Les cellules ont été ensuite infectées avec le phage auxiliaire M13 (M13K07, Biolabs, rapport bactéries / phages = 1/3) à 37°C pendant 20 min et la production de phage-Fc a été poursuivie pendant une nuit à 26°C, à 230rpm 2YT/ampicilline/glucose avec de l'IPTG 0,5 mM et 50 µg de kanamycine/ml. Le jour suivant, les phages ont été précipités avec du PEG6000 en utilisant des protocoles standards, remis en suspension dans 1 ml de tampon PBS à pH 7,4 et titrés en infectant des cellules XL1-Blue.

### 2.1 Protéines recombinantes utilisées :

Le complément C1q est disponible commercialement (Calbiochem).

CD16a est un récepteur activateur qui présente un polymorphisme V/F en position 158, sur le site de liaison au Fc. L'affinité est meilleure pour CD16aV.

CD16aV est disponible commercialement (R&D system).

CD16aF a été produit par PX'Therapeutics.

CD32a est un récepteur activateur qui présente un polymorphisme H/R en position 131, sur le site de liaison au Fc. L'affinité est meilleure pour CD32aH.

CD32aR est disponible commercialement (R&D system).

CD32aH a été produit par PX'Therapeutics. CD32b est un récepteur inhibiteur qui a une moins bonne affinité pour les IgG1 que CD32aR. Il est disponible commercialement (R&D system).

### 2.2. Sélections en phase solide :

Pour les sélections en phase solide, les phages-Fc dilués en PBS / 5% de lait écrémé / 0,1% de Tween 20 ont été incubés dans 8 puits de plaques Maxisorp (1-4×10¹¹ phages / puits dans 100µl final) préalablement revêtues avec 500ng/puits de CD16aV, CD 16a V biotinylé, CD16aF biotinylé, de C1q biotinylé, de CD32aR biotinylé ou de CD32b biotinylé et bloqués avec 5% de lait écrémé en PBS. Après une incubation de 2 heures à 37°C, les puits ont été lavés 8 fois avec du PBS/0,1% Tween 20 et 2 fois avec du PBS. Pour les sélections sur CD32aR et CD32b, des tours de contre-sélection ont aussi été réalisés : avant l'étape de liaison sur la cible immobilisée sur plaque Maxisorp, les phages-Fc ont été pré-incubés de manière similaire sur 8 puits avec le récepteur compétiteur. Seuls les phages-Fc non liés sur la première cible ont ensuite été transférés sur les puits contenant la deuxième cible comme décrit précédemment. Les phages sélectionnés ont ensuite été élués par infection avec des bactéries XL1-Blue en phase de croissance exponentielle (2×150µl/puits, 20 min. à 37°C sans agitation). Les bactéries infectées ont ensuite été étalées sur milieu solide 2YT/ampicilline/glucose. Le jour suivant, les cellules ont été resuspendues en milieu 2YT avec 15% de glycérol, congelées et conservées à -80°C jusqu'au tour de sélection suivant.

### 2.3. Sélections en phase liquide :

Pour les sélections en phase liquide, 4×10¹¹ phages ont été d'abord incubés avec du CD16aV biotinylé (250 nM), du CD16aF biotinylé (1000 nM), ou avec du C1q biotinylé (250nM) pendant 1 heure à température ambiante sous faible agitation. Des billes magnétiques revêtues de streptavidine (Dynal) préalablement bloquée avec 5% de lait écrémé en PBS ont ensuite été ajoutées aux phages pendant 30 minutes à température ambiante. Les complexes phage-billes ont été lavés 10 fois avec du PBS/0,1% Tween 20 en utilisant un aimant. Les complexes phages-billes ont ensuite été utilisés pour infecter 5 ml de bactéries XL1-Blue en croissance exponentielle, qui ont été étalées sur milieu solide 2YT/ampicilline/glucose. Le jour suivant, les cellules ont été resuspendues en milieu 2YT avec 15% de glycérol, congelées et conservées à -80°C jusqu'au tour de sélection suivant.

### 2.4. Sélections à partir des banques :

### Banque Mut3 :

Pour les sélections en phase liquide, 6 tours ont été réalisées sur 3 cibles biotinylées : CD16aV, CD16aF et C1q (clones notés DL6A, DL6B et QL6A). Pour les tours de sélections en phase solide, 6 tours ont été réalisés sur 3 cibles : CD16aV, CD16aV biotinylé et C1q biotinylé (clones notés DS6A, DS6B et QS6A).

### Banque Mut4sel :

Les tours de sélection ont été réalisés en phase solide uniquement : 3 tours de sélection sur CD16aV-biotinylé, 3 tours sur C1q-biotinylé, 3 tours sur CD32aR-biotinylé (+/déplétion sur CD32b pour le 3^{ème} tour) et 3 tours sur CD32b-biotinylé (+/- déplétion sur CD32aR pour le 3^{ème} tour), (clones notés A3A, G3A, J3A/B et K3A/B).

### Banque Mut5 :

Les tours de sélection ont été réalisés comme précédemment : 3 tours de sélection sur CD16aF-biotinylé, 3 tours sur C1q-biotinylé, 3 tours sur CD32aR-biotinylé, 3 tours sur CD32b-biotinylé, 2 tours sur CD32aR-biotinylé et 2 tour avec déplétion sur CD32b-biotinylé, et 2 tours sur CD32b-biotinylé et 2 tours de déplétion sur CD32aR-biotinylé (clones notés N3A, O3A, P3A, Q3A, P4B, et Q4B).

La sélection est ensuite faite comme précédemment décrit.

### 2.5. Clonage en pool dans le vecteur pMGM05 :

Les clones sélectionnés à l'issue des tours de sélection ont ensuite été directement transférés en mélange (environ 10⁴ clones par condition) dans le vecteur eucaryote pMGM05-CD20 (pCEP4 InvitroGen), qui contient les mêmes sites de clonage que le phagemide pMG58 pour le fragment Fc (BamHI et NotI) et la chaîne variable VH de l'anticorps anti-CD20. Cette construction entraîne la mutation de deux acides aminés dans le Fc (aa224 et 225, HT changé en GS) et l'ajout de la séquence EFAAA en C-terminal du Fc, mais permet de tester très rapidement un très grand nombre de clones. Dans un premier temps, il a été vérifié que ces mutations ne modifiaient pas la liaison de l'IgG-WT aux différents récepteurs. Par la suite, les contrôles positifs ont été clonés dans ce système afin de le valider :
- IgG1-S239D, I332E, provenant de l'anticorps anti-CD19 XmAb5574 de Xencor (C1) : témoin positif pour CD16a ;
- IgG1-G236A, provenant de Xencor (C4) : témoin positif pour CD32aH/R ;
- IgG1-K326W, E333S, provenant d'Abgenix/Genentech (C3) : témoin positif pour C1q ; et
- IgG1-S267E, L328F, provenant de l'anticorps anti-CD19 XmAb5574 de Xencor (C5) : témoin positif pour CD32b.

L'ADN d'une centaine de clones isolés par tour de sélection a été séquencé par PCR sur colonies. Après analyses bioinformatiques, les clones comportant des mutations nouvelles ont été congelés à -80°C en bactérie XL1-Blue et les séquences inclues dans notre base de données. Ainsi, 158 clones ont été isolés de la banque Mut3, 371 clones de la banque Mut4sel et 171 clones de la banque Mut5.

### 2.6. Production des variants en cellules HEK293 :

La chaîne légère de l'anti-CD20 a été insérée dans un vecteur pCEP4 identique au vecteur utilisé pour la chaîne lourde, noté pMGM01-CDC20 (pCEP4 InvitroGen). Des cellules HEK293-F Freestyle^{™} (Invitrogen), cultivées dans des plaques à 24 puits, ont été co-transfectées avec les vecteurs pMGM01-CD20 et pMGM05-CD20 (Fc-WT et variants) dans des quantités équimolaires (250 ng/ml) avec un réactif Freestyle^{™} MAX (1 µl/ml) en utilisant des protocoles standard (Invitrogen). Les cellules ont été cultivées en suspension dans du milieu exempt de sérum pendant 7 jours post-transfection et les surnageants (1 ml) contenant des IgG ont été récoltés après centrifugation des cellules à 100 g pendant 10 min. Les IgG sécrétées dans les surnageants ont été quantifiées en utilisant un test ELISA sur la protéine recombinante L (Pierce), avec un anticorps anti-CD20 purifié produit dans des cellules 293-F utilisées en tant que standard. Les surnageants et les anticorps standard, dilués en série dans du PBS/0,05% Tween-20, ont été testés sur immunoplaques Maxisorp (Nunc) préalablement enduites de 0,25 µg protéine L/puits et bloquées avec 5% de lait écrémé dans du PBS. Après incubation pendant 1 heure à 37°C, les puits ont été lavés trois fois avec du PBS/0,05% Tween-20. Des variants IgG liés ont été détectés avec un fragment F(ab')2 d'IgG HRP de chèvre anti-humaine (spécifique de la chaîne y) (Sigma). Les variants d'IgG produits ont été quantifiés (1-4µg / ml) en utilisant la courbe standard.

### 2.7. Tests ELISA de variants IgG produits dans les surnageants de cellules 293-F :

Les variants d'IgG ont été testés pour leur liaison au complément C1q humain et plusieurs FcR humains par ELISA. Des immunoplaques Maxisorp ont été revêtues avec 0,5 µg C1q complément / puits, 0,05 µg CD32aH / puits, 0,2 µg CD16aF / puits ou 0,1 µg CD 16a V / puits dans du PBS. Des plaques chélatantes au nickel immobilisantes (Nunc) ont été revêtues avec 0,1 µg CD32aR/puits ou 0.4µg CD32b/puits dans 0,01 M de KCl. Après le revêtement pendant une nuit à 4°C, les plaques ont été lavées deux fois avec du PBS/0,05% Tween-20 et saturées avec du PBS/4% de BSA pendant 2 heures à 37°C. En parallèle, les surnageants ont été dilués dans du PBS à une concentration finale de 0,5 µg d'IgG/ml et mélangés avec des F(ab')2 d'IgG HRP de chèvre anti-humaine à la même concentration pendant 2 heures à température ambiante. Les IgG agrégées aux F(ab')2 sont ensuite incubées sous agitation douce pendant 1 heure à 30°C sur les plaques ELISA saturées sans dilution pour C1q, CD16aF, CD32aR et CD32b (i.e. IgG à 0,5 µg/ml), diluées dans du PBS à 0,25 µg/ml pour CD16aV et CD32aH. Les plaques sont ensuite révélées avec TMB (Pierce) et l'absorbance lues à 450 nm.

### Sélections sur la banque Mut3 :

Grâce à ce test ELISA, les variants sélectionnés par phage display ont été testés pour leur liaison au complément C1q et aux différents récepteurs. Ils ont été testés en comparaison avec le type sauvage Fc (Fc-WT) et les témoins positifs. 36 clones positifs sur CD16aV et/ou C1q ont été ainsi sélectionnés sur la banque Mut3 et utilisés pour construire la banque Mut4.

### Sélections sur la banque Mut4sel :

Les tests ELISA réalisés sur les 371 clones isolés ont permis d'identifier 116 clones avec un ratio supérieur à 2 pour au moins un FcyR et 17 clones avec un ratio supérieur à 3 pour le C1q uniquement, ce qui correspond donc à 133 clones d'intérêt (Tableau 1).

**Tableau 1 : 133 clones d'intérêt isolés**

| **Nom du mutant** | **Mutations** | **Résultats tests ELISA** | | | |
|---|---|---|---|---|---|
| | | **C1q** | **CD16aV** | **CD32aR** | **CD32 b** |
| K3B-01 | N315D, N361H, P396L | 3,46 | 2,01 | 7,65 | 7,30 |
| A3A-105 | G316D, K326E | 5,18 | 2,48 | 4,50 | 7,13 |
| G3A-59 | N315D, T350A, P396L | 4,05 | 2,22 | 7,95 | 6,19 |
| J3B-81 | V284L, V323I, P352S, A378V, Y436H | 4,32 | 2,52 | 13,17 | 6,12 |
| J3A-123 | V266M, P352S, A378V | 0,77 | 0,35 | 7,76 | 5,93 |
| J3B-118 | P396L, N421T | 3,88 | 2,58 | 9,00 | 4,99 |
| A3A-46 | T350A, P396L | 2,19 | 2,40 | 6,17 | 4,79 |
| J3A-129 | T350A, G385R, P396L | 1,33 | 2,84 | 6,56 | 4,33 |
| QL4A-55 | N315D, P396L | 2,06 | 2,00 | 3,41 | 4,27 |
| A3A-27 | K326T, H435R | 5,81 | 2,21 | 3,41 | 4,10 |
| A3A-123 | N315D, P396L, K447N | 4,30 | 2,70 | 4,53 | 4,08 |
| J3A-109 | N315D, P396L, N434K | 1,75 | 2,46 | 6,01 | 4,07 |
| J3B-124 | P396L | 2,25 | 2,52 | 7,78 | 3,86 |
| A3A-79 | E283G, A378T, V397M | 1,62 | 1,35 | 3,07 | 3,83 |
| G3A-69 | K326T, V397M, F423L, A431V | 3,30 | 2,51 | 5,38 | 3,43 |
| A3A-178 | P396L, F423S | 4,03 | 1,73 | 3,55 | 3,37 |
| DS3A-39 | V284L, A378V | 2,76 | 3,18 | 3,48 | 3,30 |
| J3A-89 | V284L, N325S, A378V | 0,80 | 0,51 | 4,50 | 3,29 |
| DS3A-09 | A378V | 1,30 | 3,03 | 2,70 | 3,21 |
| J3A-120 | N315D, P343S, P396L | 1,66 | 2,32 | 4,71 | 3,16 |
| K3A-35 | K290E, S375R, F405V | 1,89 | 1,64 | 3,93 | 3,12 |
| DL3A-58 | S375R | 1,85 | 2,25 | 3,95 | 3,11 |
| A3A-50 | V284L, K322E, A378V | 0,67 | 1,50 | 5,08 | 3,10 |
| DL4A-146 | P352S, A378V | 1,56 | 2,31 | 2,35 | 3,10 |
| A3A-91 | K340E, A378V, N389S, N421T | 2,39 | 1,88 | 2,67 | 3,03 |
| DS3A-93 | F243I, P352S, A378V | 1,29 | 1,71 | 4,19 | 2,97 |
| A3A-184 | K334N, P352S, V397M | 1,44 | 2,90 | 3,06 | 2,91 |
| A3A-112 | T307P, N389T, V397M, S442F | 3,17 | 1,74 | 2,39 | 2,91 |
| A3A-132 | K248E, N315D, P396L | 5,03 | 2,12 | 3,70 | 2,79 |
| DS3B-92 | V284L, Y349H, A378V | 2,52 | 1,98 | 2,57 | 2,79 |
| QL3A-61 | N286I, A378V | 1,33 | 2,07 | 2,20 | 2,77 |
| J3B-115 | T359A, S383R, V397M | 1,72 | 1,69 | 5,17 | 2,77 |
| A3A-173 | K248E, K334R, A378V | OVER (>30) | 1,63 | 2,96 | 2,76 |
| A3A-164 | A378V, K447N | 1,64 | 2,41 | 2,82 | 2,71 |
| G3A-118 | N286I, P352S, A378V | 1,21 | 2,45 | 4,83 | 2,68 |
| QS5A-66 | T394P | 2,64 | 1,81 | 2,05 | 2,67 |
| DL4A-55 | A378V, N421T | 1,20 | 1,87 | 2,34 | 2,66 |
| G3A-176 | V259A, V284L, A378V, N421T | 1,05 | 1,68 | 2,10 | 2,61 |
| G3A-09 | A378T, V397M, K447N | 1,58 | 2,23 | 3,12 | 2,45 |
| DL3 A-131 | A378T, V397M | 1,20 | 1,72 | 2,30 | 2,40 |
| J3A-10 | T307P, P396L | 5,18 | 2,74 | 5,71 | 2,39 |
| J3B-49 | N315D, T393A, P396L | 1,42 | 1,77 | 5,17 | 2,30 |
| K3A-41 | V302A, P352L, L398P | 1,11 | 0,77 | 2,17 | 2,30 |
| DL4A-54 | P352L, Q418P | 1,35 | 1,49 | 1,58 | 2,30 |
| A3A-06 | K248E, A378T, V397M | 4,79 | 1,59 | 1,78 | 2,29 |
| G3A-154 | A378T, V397M, F423P, S442P | 2,51 | 2,11 | 4,14 | 2,28 |
| A3A-12 | V363I, V397M | 3,02 | 1,56 | 2,99 | 2,27 |
| J3A-14 | K326T | 1,83 | 1,43 | 4,25 | 2,26 |
| G3A-05 | V323I, S383N | 2,21 | 1,33 | 3,37 | 2,25 |
| J3B-138 | K334R, T394P | 4,41 | 1,09 | 5,34 | 2,24 |
| QL2A-11 | V302A | 0,97 | 0,68 | 1,52 | 2,22 |
| K3B-32 | S254F, A378T, V397M | 5,80 | 1,42 | 3,72 | 2,22 |
| G3A-88 | K320E, T394P, G402D | 1,32 | 1,88 | 4,94 | 2,21 |
| K3B-30 | I253F, K326T, F423L, A431V | 7,34 | 1,58 | 5,99 | 2,20 |
| A3A-176 | V284A, A378V, A431T | 3,32 | 1,82 | 2,01 | 2,20 |
| K3A-59 | N315H, Y319H, V323I | 2,88 | 0,59 | 3,33 | 2,17 |
| J3B-120 | A378V, L398P | 1,29 | 1,67 | 2,94 | 2,17 |
| QL4B-10 | S383R, V397M | 1,40 | 1,35 | 1,95 | 2,08 |
| J3B-89 | K290E, V308I, A327T, S383N | 0,50 | 0,14 | 0,30 | 2,05 |
| G3A-83 | C226Y, A378V, N421T | 0,90 | 2,26 | 2,00 | 2,04 |
| G3A-165 | V323I, T393I | 1,33 | 1,52 | 4,15 | 2,04 |
| J3B-135 | K248E, S383R, V397M, N434S | 6,36 | 1,51 | 3,99 | 2,04 |
| A3A-137 | K290E, V323I, F405L | 1,91 | 1,65 | 3,02 | 2,03 |
| K3B-94 | R255W, A287T, P352S, A378V | 4,66 | 1,52 | 5,08 | 2,02 |
| A3A-30 | V284L, T350A | 2,09 | 1,47 | 2,47 | 2,01 |
| K3A-07 | N315D, A378V | 1,30 | 1,76 | 2,26 | 2,01 |
| G3A-164 | T394P, N434S | 1,72 | 1,19 | 2,29 | 1,99 |
| J3A-16 | N286Y, P352S, A378V | 1,46 | 1,83 | 3,54 | 1,98 |
| G3A-43 | A231V, A378V | 1,13 | 2,44 | 3,57 | 1,96 |
| A3A-09 | V308G, V323I | 3,65 | 1,23 | 2,26 | 1,95 |
| G3A-106 | S254F, V284L, A378V | 1,57 | 1,95 | 3,96 | 1,94 |
| J3A-08 | K274R, A378V, N421T | 1,17 | 1,53 | 3,47 | 1,94 |
| QL3A-20 | V397M | 2,49 | 1,96 | 2,60 | 1,93 |
| G3A-108 | T394P, K414R | 1,50 | 1,54 | 3,67 | 1,91 |
| G3A-163 | V323I, M428T | 1,06 | 1,05 | 2,41 | 1,89 |
| A3A-125 | T394P, K447N | 1,94 | 1,83 | 2,48 | 1,84 |
| J3B-109 | E345G, V397M | 1,18 | 1,36 | 2,37 | 1,83 |
| DS3B-33 | K326T, F423L, A431V | 5,29 | 1,31 | 1,73 | 1,80 |
| QL4A-28 | F243L, P247T, Q362R, G402D, S440N | 0,74 | 1,65 | 2,88 | 1,80 |
| J3B-101 | Y278H, N315D, P396L | 1,18 | 1,64 | 2,35 | 1,78 |
| A3A-140 | D312G, A378T, V397M | 1,47 | 1,39 | 2,23 | 1,76 |
| G3A-25 | V262A, V305A, A378V | 1,76 | 1,56 | 2,01 | 1,70 |
| G3A-45 | K246R, A378V | 1,15 | 2,46 | 2,76 | 1,68 |
| QS6A-78 | V323I | 1,41 | 1,31 | 2,03 | 1,68 |
| K3B-91 | V308I, K326T, F423L, A431V | 4,84 | 1,13 | 2,71 | 1,65 |
| G3A-103 | K248E, A378V | 2,54 | 2,00 | 1,96 | 1,64 |
| G3A-07 | N276S, T394P, K439Q | 2,27 | 1,13 | 2,17 | 1,59 |
| A3A-17 | K290E, E380G | 1,64 | 2,02 | 1,91 | 1,59 |
| J3B-16 | S267G, A378T, V397M | 0,85 | 0,57 | 4,95 | 1,58 |
| J3B-23 | N286I, A378V, F423Y | 1,13 | 1,81 | 3,33 | 1,57 |
| J3B-68 | K320E, T350A | 0,82 | 1,57 | 3,30 | 1,55 |
| A3A-37 | A231T, K290E, S383N, F423L | 1,41 | 1,36 | 2,02 | 1,53 |
| J3A-49 | K320R, A378T, V397M | 0,60 | 0,45 | 2,15 | 1,52 |
| A3A-31 | K334R, L410R | 6,23 | 1,36 | 1,57 | 1,50 |
| A3A-41 | V323I, P352L, L398P | 2,00 | 1,46 | 2,53 | 1,47 |
| K3A-36 | K248E, K320M | 2,81 | 1,69 | 2,34 | 1,42 |
| J3A-06 | A378T, V397M, V412M | 1,60 | 1,65 | 2,74 | 1,41 |
| G3A-139 | T307N, V323I | 1,30 | 1,10 | 2,57 | 1,41 |
| G3A-98 | S375R, N434S | 1,14 | 1,56 | 3,02 | 1,40 |
| K3B-80 | V284L, T366A, A378V | 2,58 | 1,25 | 2,27 | 1,39 |
| J3B-74 | P230S, N389S, T394P | 0,94 | 0,65 | 1,81 | 1,39 |
| K3B-41 | Y349S, V397M | 1,82 | 1,27 | 2,11 | 1,38 |
| G3A-13 | K248E, A339T, T350A, S440N | 2,10 | 1,28 | 2,89 | 1,37 |
| J3B-44 | K246E, K274E, V397M | 1,59 | 1,05 | 2,38 | 1,37 |
| K3B-43 | A231P, K290E, S383N | 1,66 | 1,43 | 2,15 | 1,36 |
| J3B-107 | T307P, A378T, N389T | 1,50 | 1,32 | 2,05 | 1,36 |
| K3B-33 | K334R, K392R, S440G | 4,87 | 0,77 | 0,97 | 1,33 |
| K3A-11 | K246E, K290E, T307P, N389T | 1,73 | 1,65 | 2,00 | 1,33 |
| K3B-49 | D376G, S383R, V397M | 2,54 | 1,34 | 2,57 | 1,33 |
| G3A-95 | I336T, A378V | 1,05 | 2,25 | 1,82 | 1,31 |
| K3B-90 | V308A, K334R, A378V, K447N | 1,32 | 1,30 | 3,72 | 1,27 |
| A3A-146 | K290E, R355Q, S383N | 1,49 | 1,38 | 2,15 | 1,26 |
| A3A-11 | S254F, K447N | 4,78 | 1,18 | 1,02 | 1,22 |
| G3A-31 | K248E, K334Q | 3,60 | 1,52 | 1,06 | 1,21 |
| QL2A-16 | K248E, N421T | 5,30 | 0,79 | 0,77 | 1,19 |
| J3A-43 | K248E, P352L, Q418P | 2,96 | 1,23 | 2,13 | 1,19 |
| J3A-28 | E333G, A378T, V397M | 1,89 | 1,83 | 1,73 | 1,18 |
| A3A-07 | L365P, T366S, A378T | 1,20 | 1,94 | 1,17 | 1,18 |
| A3A-96 | K248E, L365P, A378T | 4,09 | 1,43 | 0,70 | 1,12 |
| K3B-87 | V323I, F405L | 1,56 | 0,93 | 2,36 | 1,10 |
| G3A-148 | K248E, S375R | 3,79 | 1,25 | 1,64 | 1,08 |
| K3B-34 | K334N, T394P, S408N, K414N | 0,62 | 1,18 | 2,25 | 1,02 |
| K3B-89 | L309M, A378V | 1,98 | 1,42 | 2,07 | 1,02 |
| G3A-49 | T307A, E380G | 3,66 | 0,91 | 0,82 | 1,02 |
| G3A-159 | E382G, L432P, Q438DEL | 3,00 | 0,91 | 1,13 | 0,98 |
| G3A-28 | T307P, H435R | 3,49 | 1,06 | 0,95 | 0,96 |
| K3B-78 | H310R, P352L, Q418P | 1,20 | 0,84 | 2,04 | 0,96 |
| G3A-22 | P352S, E382G, L432P, Y436N | 3,11 | 0,95 | 0,62 | 0,94 |
| G3A-56 | K248E, N421T, K447N | 3,25 | 0,71 | 1,03 | 0,89 |
| G3A-145 | E430G, K447N | 4,34 | 1,01 | 0,81 | 0,88 |
| A3A-21 | S267N, N384D, N389S, P396L | 4,32 | 1,00 | 0,56 | 0,88 |
| G3A-33 | K248E, S267N, I336M, P352L, P396L | 5,67 | 0,79 | 0,39 | 0,76 |
| G3A-51 | V240I, K246E, P353L | 3,90 | 0,95 | 0,51 | 0,64 |

Parmi ces clones, 47 clones améliorés ont été utilisés pour construire la banque Mut5. A partir de ces résultats, 18 nouveaux variants ont été construits par mutagenèse dirigée de manière à cumuler des mutations d'intérêt (Tableau 2).

**Tableau 2 : 18 nouveaux variants construits et comparés aux variants de référence**

| *NA = Non Déterminé* | | | | | | | |
|---|---|---|---|---|---|---|---|
| **Nom du mutant** | **Mutations** | **Résultats tests ELISA** | | | | | |
| | | **C1q** | **CD16aF** | **CD16aV** | **CD32aH** | **CD32aR** | **CD32b** |
| A3A-105 | G316D, K326E | 5,18 | 1,31 | 2,48 | 1,50 | 4,50 | 7,13 |
| A3A-105A | G316D, K326E, T394P | 15,33 | 1,09 | 1,45 | 0,66 | 5,60 | 1,21 |
| A3A-105B | G316D, K326E, P396L | 14,30 | 2,25 | 3,52 | 3,92 | 12,64 | 4,16 |
| A3A-105C | G316D, K326E, V397M | 9,61 | 2,90 | 1,86 | 1,81 | 11,00 | 2,22 |
| A3A-105D | G316D, K326E, A378V | 13,32 | 2,46 | 3,68 | 1,39 | 8,20 | 5,96 |
| A3A-184 | K334N, P352S, V397M | 1,44 | 1,71 | 2,90 | 1,34 | 3,06 | 2,91 |
| A3A-184A | K334N, P352S, A378V, V397M | 1,75 | 3,40 | 4,74 | 3,25 | 5,03 | 2,32 |
| DS3A-09 | A378V | 1,30 | 3,15 | 3,03 | 2,90 | 2,70 | 3,21 |
| DS3A-09A | A378V, T394P | 1,24 | 2,29 | 2,83 | 2,26 | 2,00 | 1,26 |
| DS3A-09B | A378V, P396L | NA | 1,83 | 2,43 | NA | NA | NA |
| DS3A-09C | A378V, V397M | NA | 1,48 | 2,25 | NA | NA | NA |
| G3A-43 | A231V, A378V | 1,13 | 1,79 | 2,44 | 1,42 | 3,57 | 1,96 |
| G3A-43A | A231V, A378V, T394P | NA | 1,13 | 1,65 | NA | NA | NA |
| G3A-43B | A231V, A378V, P396L | NA | 1,85 | 2,42 | NA | NA | NA |
| G3A-43C | A231V, A378V, V397M | NA | 1,81 | 2,68 | NA | NA | NA |
| G3A-45 | K246R, A378V | 1,15 | 1,92 | 2,46 | 1,38 | 2,76 | 1,68 |
| G3A-45A | K246R, A378V, T394P | NA | 1,34 | 2,34 | NA | NA | NA |
| G3A-45B | K246R, A378V, P396L | NA | 1,37 | 1,75 | NA | NA | NA |
| G3A-45C | K246R, A378V, V397M | NA | 1,51 | 2,21 | NA | NA | NA |
| G3A-95 | I336T, A378V | 1,05 | 1,29 | 2,25 | 1,17 | 1,82 | 1,31 |
| G3A-95A | I336T, A378V, T394P | NA | 1,50 | 1,90 | NA | NA | NA |
| G3A-95B | I336T, A378V, P396L | NA | 2,00 | 2,54 | NA | NA | NA |
| G3A-95C | I336T, A378V, V397M | NA | 1,55 | 2,01 | NA | NA | NA |
| J3B-118 | P396L, N421T | 3,88 | 1,26 | 2,58 | NA | 9,00 | 4,99 |
| J3B-118A | A378V, P396L, N421T | NA | 1,76 | 3,37 | NA | NA | NA |
| QL3A-20 | V397M | 2,49 | 0,62 | 1,96 | 1,09 | 2,60 | 1,93 |
| QS5A-66 | T394P | 2,64 | NA | 1,81 | NA | 2,05 | 2,67 |

Finalement, à partir de ces 151 variants testés, 26 variants d'intérêt ont été sélectionnés pour être produits à plus grande échelle et étudiés plus précisément (Tableau 3).

**Tableau 3 : 26 nouveaux variants sélectionnés**

| *NA = Non Déterminé* | | | | | | | |
|---|---|---|---|---|---|---|---|
| **Nom du mutant** | **Mutations** | **Résultats tests ELISA** | | | | | |
| | | **C1q** | **CD16 aF** | **CD16a V** | **CD32a H** | **CD32a R** | **CD32 b** |
| A3A-105 | G316D, K326E | 5,18 | 1,31 | 2,48 | 1,50 | 4,50 | 7,13 |
| A3A-105D | G316D, K326E, A378V | 13,32 | 2,46 | 3,68 | 1,39 | 8,20 | 5,96 |
| A3A-14 | S298N, A378V | 0,53 | 0,96 | 0,85 | 0,22 | 0,35 | 0,88 |
| A3A-173 | K248E, K334R, A378V | NA | NA | 1,63 | NA | 2,96 | 2,76 |
| A3A-184 | K334N, P352S, V397M | 1,44 | 1,71 | 2,90 | 1,34 | 3,06 | 2,91 |
| A3A-184A | K334N, P352S, A378V, V397M | 1,75 | 3,40 | 4,74 | 3,25 | 5,03 | 2,32 |
| A3A-31 | K334R, L410R | 6,23 | NA | 1,36 | NA | 1,57 | 1,50 |
| DL4A-54 | P352L, Q418P | 1,35 | 1,18 | 1,49 | 1,09 | 1,58 | 2,30 |
| G3A-103 | K248E, A378V | 2,54 | 1,17 | 2,00 | 0,83 | 1,96 | 1,64 |
| G3A-139 | T307N, V323I | 1,30 | 0,81 | 1,10 | 0,65 | 2,57 | 1,41 |
| G3A-43 | A231V, A378V | 1,13 | 1,79 | 2,44 | 1,42 | 3,57 | 1,96 |
| G3A-45 | K246R, A378V | 1,15 | 1,92 | 2,46 | 1,38 | 2,76 | 1,68 |
| G3A-88 | K320E, T394P, G402D | 1,32 | NA | 1,88 | NA | 4,94 | 2,21 |
| G3A-95 | I336T, A378V | 1,05 | 1,29 | 2,25 | 1,17 | 1,82 | 1,31 |
| J3A-06 | A378T, V397M, V412M | 1,60 | 1,24 | 1,65 | 1,58 | 2,74 | 1,41 |
| J3A-14 | K326T | 1,83 | 0,52 | 1,43 | 0,99 | 4,25 | 2,26 |
| J3A-16 | N286Y, P352S, A378V | 1,46 | 1,35 | 1,83 | 1,77 | 3,54 | 1,98 |
| J3A-28 | E333G, A378T, V397M | 1,89 | 1,41 | 1,83 | 1,39 | 1,73 | 1,18 |
| J3B-115 | T359A, S383R, V397M | 1,72 | 1,32 | 1,69 | 1,42 | 5,17 | 2,77 |
| J3B-118 | P396L, N421T | 3,88 | 1,26 | 2,58 | NA | 9,00 | 4,99 |
| J3B-118A | A378V, P396L, N421T | NA | 1,76 | 3,37 | NA | NA | NA |
| J3B-16 | S267G, A378T, V397M | 0,85 | 0,59 | 0,57 | 0,42 | 4,95 | 1,58 |
| J3B-23 | N286I, A378V, F423Y | 1,13 | 1,91 | 1,81 | 1,57 | 3,33 | 1,57 |
| K3B-01 | N315D, N361H, P396L | 3,46 | 0,98 | 2,01 | 2,10 | 7,65 | 7,30 |
| K3B-90 | V308A, K334R, A378V, K447N | 1,32 | 1,06 | 1,30 | 1,40 | 3,72 | 1,27 |
| QL2A-16 | K248E, N421T | 5,30 | 0,69 | 0,79 | 0,33 | 0,77 | 1,19 |

### Sélections sur la banque Mut5 :

Les tests ELISA réalisés sur les 171 clones isolés ont permis d'identifier 87 clones avec un ratio supérieur à 2 pour au moins l'un des FcyRs testés ou le complément C1q (Tableau 4).

**Tableau 4 : 87 clones avec ratio supérieur à 2**

| **Nom du mutant** | **Mutations** | **Résultats tests ELISA** | | | | | |
|---|---|---|---|---|---|---|---|
| | | **C1q** | **CD16a F** | **CD16aV** | **CD32a H** | **CD32aR** | **CD32 b** |
| P3A-01 | K334N, S383R | 0,93 | 1,34 | 2,05 | 1,10 | 2,00 | 1,57 |
| P3A-17 | K334N, Y373D, A378V | 0,88 | 1,46 | 1,63 | 1,35 | 2,17 | 1,55 |
| P3A-18 | K248E, N286Y, K334R, A378V, K447N | 24,7 7 | 0,94 | 1,62 | 1,07 | 1,24 | 1,13 |
| P3A-23 | N361H, P396L | 2,70 | 1,75 | 2,49 | 2,53 | 3,33 | 1,76 |
| P3A-30 | K288E, K334N, P352S, V397M | 1,18 | 1,35 | 4,33 | 2,14 | 1,79 | 1,41 |
| P3A-31 | K248E, T359A, S383R, V397M | 5,80 | 0,80 | 2,05 | 1,80 | 1,62 | 1,51 |
| P3A-50 | T307P, A378V | 1,11 | 1,55 | 2,89 | 2,20 | 1,85 | 1,44 |
| P3A-56 | G316D | 1,74 | 1,07 | 1,90 | 2,02 | 1,45 | 1,39 |
| P3A-70 | K274R, A378V, V397M | 1,13 | 1,93 | 4,05 | 3,54 | 4,89 | 3,20 |
| P3A-74 | V302A, V397M | 0,92 | 0,72 | 0,63 | 1,47 | 2,43 | 1,32 |
| P3A-94 | L309P, T359A, S383R, V397M | 1,02 | 1,32 | 2,11 | 2,54 | 1,79 | 1,32 |
| N3A-111 | V302A, K334N, S375R | 1,36 | 0,82 | 1,18 | 1,20 | 2,73 | 2,33 |
| N3A-113 | E283G, Y349S, P396L | 6,55 | 1,30 | 1,73 | 2,17 | 5,19 | 2,94 |
| N3A-114 | K320E, S375R, F405V | 0,69 | 1,03 | 1,12 | 0,92 | 2,11 | 1,60 |
| N3A-117 | K334N | 1,32 | 1,61 | 2,28 | 1,31 | 1,93 | 1,98 |
| N3A-123 | K326E | 3,15 | 1,03 | 2,21 | 1,27 | 7,71 | 3,53 |
| N3A-132 | S375R, N389S | 1,71 | 0,99 | 1,10 | 1,41 | 3,50 | 2,35 |
| N3A-133 | K334N, P352S, K447N | 0,77 | 1,02 | 2,23 | 0,88 | 1,25 | 1,40 |
| N3A-138 | S298T, K334R, K370E | 3,56 | 0,90 | 0,68 | 0,59 | 0,73 | 1,17 |
| N3A-14 | T307N, A378V, K414R | 1,00 | 1,70 | 2,83 | 2,50 | 2,49 | 1,74 |
| N3A-141 | T350A, V379A, G385R, P396L | 2,82 | 1,30 | 1,79 | 2,36 | 5,60 | 5,70 |
| N3A-145 | S267G, K290E, E293G, E380G, V397M | 0,99 | 0,70 | 0,38 | 0,31 | 2,45 | 1,33 |
| N3A-150 | S267G, K334N, P352S, V397M | 0,86 | 0,76 | 0,68 | 0,33 | 4,82 | 1,78 |
| N3A-161 | K248E, N315D, A378V | 8,59 | 1,28 | 2,49 | 1,12 | 1,23 | 1,32 |
| N3A-175 | S375R, F405V | 0,94 | 1,16 | 2,47 | 1,99 | 1,30 | 1,10 |
| N3A-177 | V282A, K334R, | 5,95 | 1,15 | 1,28 | 1,68 | 3,22 | 1,68 |
| | T394P | | | | | | |
| N3A-190 | T307P, T394P | 2,46 | 1,35 | 2,46 | 2,93 | 3,33 | 1,94 |
| N3A-27 | K290E, A378V, K392R, S440G | 1,67 | 2,25 | 3,77 | 2,05 | 2,59 | 1,49 |
| N3A-28 | T366A, A378V | 1,04 | 1,77 | 2,89 | 2,26 | 1,84 | 1,36 |
| N3A-32 | K326T, K334N, P352S, N421T | 1,61 | 2,47 | 4,35 | 1,59 | 3,24 | 2,04 |
| N3A-52 | K326T, S383R, V397M | 3,70 | 1,11 | 2,07 | 1,92 | 5,40 | 2,41 |
| N3A-58 | V284L, K334R, A378V, K447N | 5,37 | 1,02 | 1,67 | 2,01 | 4,00 | 1,67 |
| N3A-59 | N315D, T366A, G385R, P396L | 1,08 | 1,25 | 1,66 | 1,75 | 2,73 | 1,56 |
| N3A-61 | V308A, K334R, A378V | 2,07 | 1,10 | 1,57 | 1,11 | 2,87 | 2,67 |
| N3A-74 | K288R, T394P | 1,38 | 1,22 | 1,51 | 1,58 | 2,15 | 1,73 |
| N3A-85 | K334R, V397M | 4,96 | 1,11 | 1,45 | 1,89 | 3,75 | 2,77 |
| N3A-87 | K290E, K320E, T350A, P396L | 0,91 | 2,15 | 2,85 | 3,12 | 5,94 | 4,63 |
| N3A-93 | A378T, P396L | 1,63 | 1,84 | 2,21 | 2,55 | 3,86 | 3,06 |
| O3A-04 | A231V, T359A, S383R, V397M | 1,00 | 1,16 | 1,21 | 1,69 | 2,35 | 1,09 |
| O3A-05 | K290E, T366A, A378V | 0,99 | 2,79 | 3,12 | 2,07 | 3,34 | 1,34 |
| O3A-10 | Y300H, T394P | 0,98 | 0,76 | 0,90 | 1,81 | 2,25 | 1,17 |
| O3A-11 | R255W, A287T, P352S, A378V, N421T | 1,85 | 1,01 | 2,10 | 2,20 | 3,15 | 1,55 |
| O3A-16 | K334R, A378V, K447N | 4,30 | 1,71 | 2,51 | 2,43 | 3,98 | 1,59 |
| O3A-17 | I336V, T359A, S383R, V397M | 0,85 | 1,79 | 2,70 | 2,30 | 1,73 | 0,93 |
| O3A-22 | K334R, P396L, H435R | 23,5 5 | 1,68 | 2,16 | 2,75 | 4,79 | 1,62 |
| O3A-24 | K326T, K447N | 2,12 | 1,23 | 2,27 | 1,55 | 2,94 | 1,40 |
| O3A-25 | V308A, K334R, A378T, V397M | 11,1 2 | 1,40 | 2,07 | 2,04 | 4,16 | 1,96 |
| O3A-34 | A231V, Y349S, V397M | 0,73 | 1,40 | 1,90 | 2,34 | 2,62 | 1,28 |
| O3A-38 | K248E, N286Y, Q418P | 2,17 | 1,59 | 1,16 | 0,99 | 0,69 | 0,57 |
| O3A-40 | V308A, A378T, V397M, V412M | 0,82 | 1,65 | 2,15 | 2,09 | 2,64 | 1,03 |
| O3A-42 | S304N, A378V | 1,04 | 2,16 | 0,53 | 0,87 | 0,87 | 1,77 |
| O3A-44 | A378V, K439R | 1,95 | 2,44 | 4,40 | 3,20 | 3,13 | 1,81 |
| O3A-45 | D270N, K334R, L410R | 3,32 | 1,57 | 0,61 | 0,56 | 1,19 | 1,36 |
| O3A-46 | V302A, A378V | 0,94 | 1,15 | 1,75 | 2,36 | 2,91 | 1,37 |
| O3A-50 | K334R, A378V, N421T | 3,62 | 1,28 | 2,49 | 2,15 | 4,02 | 2,30 |
| O3A-57 | T359A, V397M | 1,50 | 1,18 | 2,27 | 2,23 | 2,78 | 1,59 |
| O3A-66 | A378T, T394P | 1,10 | 1,42 | 2,42 | 2,36 | 2,45 | 1,76 |
| O3A-67 | K248E, N361H | 2,05 | 0,89 | 1,36 | 1,06 | 1,07 | 1,76 |
| O3A-69 | K290E, A378V | 1,44 | 2,64 | 4,04 | 2,62 | 4,11 | 2,06 |
| O3A-80 | K274R, T394P, G402D, K447N | 1,26 | 1,26 | 1,81 | 2,16 | 2,32 | 1,28 |
| O3A-81 | K274R, A378T, V397M | 1,36 | 1,20 | 2,39 | 2,06 | 3,08 | 1,58 |
| O3A-86 | K248E, K290E, N361H, P396L | 24,3 4 | 2,49 | 4,89 | 2,86 | 4,19 | 2,57 |
| O3A-93 | K290E, T394P | 2,07 | 1,45 | 3,53 | 2,54 | 3,36 | 1,78 |
| P4B-89 | T307P, T366A, A378V | 0,63 | 2,99 | 4,15 | 0,70 | 1,19 | 0,87 |
| Q4B-08 | V284L, K290E, A378V | 2,43 | 1,75 | 3,47 | 2,69 | 3,58 | 1,63 |
| Q4B-15 | K274R, A378V | 1,26 | 1,04 | 2,69 | 2,61 | 1,75 | 1,03 |
| Q4B-18 | I377T, A378V, F423Y | 1,21 | 1,08 | 2,82 | 1,96 | 1,37 | 1,07 |
| Q4B-34 | K248E, K290M, V308A, P352S | 4,47 | 0,63 | 0,56 | 0,38 | 0,70 | 0,75 |
| Q4B-59 | T350A, A378T, V397I | 1,32 | 2,22 | 2,01 | 2,76 | 2,52 | 1,51 |
| Q4B-61 | K320E, T394P, V397M | 0,87 | 1,10 | 0,98 | 1,60 | 2,43 | 1,08 |
| Q4B-68 | T307P, A378V, T394P | 3,33 | 1,53 | 4,12 | 4,01 | 3,69 | 1,03 |
| Q4B-91 | K334R, A378V | 2,81 | 1,14 | 2,35 | 2,61 | 2,46 | 0,94 |
| Q3A-01 | P352L, A378V | 1,13 | 1,14 | 1,53 | 2,04 | 1,81 | 0,87 |
| Q3A-39 | N286I, P396L, N421T | 2,80 | 1,63 | 1,99 | 3,52 | 3,09 | 1,02 |
| Q3A-58 | S267G, V397M | 0,73 | 1,30 | 0,34 | 0,40 | 2,87 | 1,02 |
| Q3A-85 | P396L, N421T, K447N | 3,80 | 1,65 | 2,17 | 3,67 | 3,50 | 1,13 |
| O3A-103 | K290E, K320T, A378V | 2,11 | 1,22 | 2,08 | 2,76 | 2,41 | 1,50 |
| O3A-117 | K334R, T394P, N421S | 0,98 | 1,05 | 1,71 | 2,29 | 3,05 | 1,37 |
| O3A-119 | K334N, V397M | 1,53 | 1,04 | 1,97 | 1,45 | 2,28 | 1,18 |
| O3A-126 | P227S, V284L, A378V | 4,58 | 1,31 | 2,93 | 2,85 | 4,47 | 1,29 |
| O3A-127 | V302A, K334R, T366A, S383R, V397M | 1,53 | 1,00 | 1,24 | 2,85 | 3,56 | 1,37 |
| O3A-131 | C261R, A378T, V397M, V412M | 2,25 | 1,03 | 1,72 | 2,00 | 2,27 | 1,15 |
| O3A-137 | K248E, T350A | 0,84 | 0,66 | 0,43 | 0,68 | 2,06 | 0,97 |
| O3A-172 | K320E, T394P | 8,19 | 0,88 | 1,46 | 2,01 | 2,52 | 1,06 |
| O3A-179 | R255Q, G385R | 0,81 | 0,74 | 0,83 | 0,57 | 2,86 | 1,30 |
| O3A-186 | T307P, V397M | 1,09 | 1,41 | 2,32 | 2,17 | 3,97 | 1,48 |
| O3A-99 | N276S, N286I, T359A, S383R, V397M | 2,14 | 1,32 | 2,27 | 2,84 | 3,09 | 1,42 |

Parmi ces variants, 10 variants d'intérêt ont été sélectionnés (Tableau 5).

**Tableau 5 : 10 variants**

| **Nom du mutant** | **Mutations** | **Résultats tests ELISA** | | | | | |
|---|---|---|---|---|---|---|---|
| | | **C1 q** | **CD16a F** | **CD16a V** | **CD32aH** | **CD32a R** | **CD32b** |
| O3A-05 | K290E, T366A, A378V | 0,9 9 | 2,79 | 3,12 | 2,07 | 3,34 | 1,34 |
| P4B-89 | T307P, T366A, A378V | 0,6 3 | 2,99 | 4,15 | 0,70 | 1,19 | 0,87 |
| O3A-86 | K248E, K290E, N361H, P396L | 24, 34 | 2,49 | 4,89 | 2,86 | 4,19 | 2,57 |
| N3A-32 | K326T, K334N, P352S, N421T | 1,6 1 | 2,47 | 4,35 | 1,59 | 3,24 | 2,04 |
| O3A-44 | A378V, K439R | 1,9 5 | 2,44 | 4,40 | 3,20 | 3,13 | 1,81 |
| N3A-27 | K290E, A378V, K392R, S440G | 1,6 7 | 2,25 | 3,77 | 2,05 | 2,59 | 1,49 |
| N3A-87 | K290E, K320E, T350A, P396L | 0,9 1 | 2,15 | 2,85 | 3,12 | 5,94 | 4,63 |
| Q4B-68 | T307P, A378V, T394P | 3,3 3 | 1,53 | 4,12 | 4,01 | 3,69 | 1,03 |
| N3A-58 | V284L, K334R, A378V, K447N | 5,3 7 | 1,02 | 1,67 | 2,01 | 4,00 | 1,67 |
| Q3A-39 | N286I, P396L, N421T | 2,8 0 | 1,63 | 1,99 | 3,52 | 3,09 | 1,02 |

### 3. Production et purification des variants d'intérêt

Les variants IgG sont obtenus par mutagénèse directe dans pCEP4-WT-H-CD20. Les contrôles IgG, i.e. C1, C3, C4, C5 et sauvage (WT), sont produits avec l'allotype G1m3 (comprenant 3 mutations par rapport à G1m1,17 : (K214R/)D356E/L358M).

Les 26 variants IgG issus de la banque Mut4sel, ainsi que le sauvage, ont été produits avec l'allotype G1m1,17. Ils sont produits par incubation pendant 6-7 jours en batchs (250-300ml) de cellules 293-E (Freestyle Invitrogen) dans du milieu F17.

Une centrifugation et filtration sont ensuite réalisées.

La purification se fait sur Protein A Hi-Trap, et l'élution est faite avec un tampon citrate 25mM pH = 3.0, neutralisation et dialyse dans du TBS ou PBS avant stérilisation.

10mg de chaque contrôle IgG, i.e. C1, C3, C4, C5 et sauvage (WTG1m3 et WTGlm1,17) sont obtenus, ainsi que 2-3mg de 26 variants IgG.

Leur caractérisation montre que le poids moléculaire est conservé, et que les profils de glycosylation sont similaires pour tous les variants.

### 4. Tests des variants d'intérêt

### 4.1. Tests de liaison sur FcRn

Des cellules Jurkat-FcRn sont incubées à pH = 6.0 avec les variants IgG à différentes concentrations (0 à 1000µg/ml) et du Rituximab-Alexa.

Une cytométrie de flux est effectuée sur le Rituximab-Alexa lié.

Les résultats ne montrent aucune perte de liaison au FcRn pour tous les variants IgG.

### 4.2 Tests de liaison à l'antigène

Des cellules Raji sont incubées avec les variants IgG à 1µg/ml pendant 15 minutes à 4°C.

Les IgG liées sont révélées par liaison avec un anticorps secondaire anti-IgG humaine-PE (pendant 15 minutes à 4°C).

Les résultats montrent que la reconnaissance de l'antigène n'est pas altérée par les différentes mutations présentes sur le Fc.

Tous les variants IgG se lient à CD20 sur les cellules, de façon similaire au contrôle IgG-WT.

### 4.3 Tests ELISA de liaison au CD16aV/F

Les anticorps purifiés ont été testés en ELISA pour leur liaison au CD16F et CD16aV selon un protocole identique à celui décrit en 2.6, en diluant les anticorps à différentes concentrations.

### 4.4. Tests d'activité ADCC

Des cellules NK sont incubées avec des cellules cibles Raji exprimant CD20, en présence de différentes concentrations de variants IgG (0.005 à 5000 ng/ml).

Le niveau de LDH intracellulaire libéré par les cellules cibles lysées est mesuré.

Des cellules NK humaines ont été purifiées à partir de sang périphérique de donneurs volontaires sains par la technique de déplétion négative développée par Miltenyi. Le test ADCC comprend l'incubation de cellules NK avec des cellules cibles Raji exprimant l'antigène CD20, en présence de différentes concentrations d'anticorps anti-CD20. Après 16 heures d'incubation, la cytotoxicité induite par les anticorps anti-CD20 a été mesurée en quantifiant dans les surnageants cellulaires la lactate déshydrogénase intracellulaire (LDH) libérée par les cellules cibles lysées. Les résultats de lyse spécifique sont exprimés en pourcentage de lyse en fonction de la concentration d'anticorps. Les valeurs d'EC50 (concentration en anticorps induisant 50% de la lyse maximale induite par l'IgG-WT) et Emax (pourcentage de lyse maximale) ont été calculés en utilisant le logiciel GraphPad PRISM.

Les résultats sont présentés en Tableaux 6 et 7.

**Tableau 6 : résultats des tests ADCC**

| **Mutant** | **G3A4 3** | **G3A4 5** | **J3B-118** | **J3B16** | **A3A-184** | **WT** |
|---|---|---|---|---|---|---|
| **EC50 : Concentration d'anticorps (ng/ml) donnant 50 % de lyse d'un contrôle interne** | 1,03 | 4,38 | 1,27 | > 5545,36 | 2,35 | 12,34 |
| **Ratio [WT]/[anticorps]** | 11.98 | 2.82 | 9.72 | <0.0022 | 5.25 | 1 |

**Tableau 7a : résultats des tests ADCC**

| **Mutant** | **EC50 : Concentration d'anticorps (ng/ml) donnant 50 % de lyse maximale d'un contrôle interne** | **Ratio [WT]/[anticorps]** |
|---|---|---|
| G3A-103 | 0,31 | 33.90 |
| A3A-184A | 0,34 | 30.91 |
| J3B-23 | 0,57 | 18.44 |
| J3A-28 | 0,57 | 18.44 |
| K3B-01 | 0,69 | 15.23 |
| A3A-14 | 0,82 | 12.82 |
| G3A-95 | 0,97 | 10.84 |
| J3B-118A | 0,97 | 10.84 |
| J3A-16 | 1,27 | 8.28 |
| J3A-06 | 1,27 | 8.28 |
| A3A-105D | 1,27 | 8.28 |
| G3A-139 | 2,80 | 3.75 |
| QL2A-16 | 4,36 | 2.41 |
| J3A-14 | 7,39 | 1.42 |
| G3A-88 | 8,07 | 1.30 |
| WT | 10,51 | 1 |
| K3B-87 | 16,33 | 0.64 |
| DL4A-54 | >5000 | <0.002 |
| A3A-90 | >5000 | <0.002 |
| A3A-34 | >5000 | <0.002 |

**Tableau 7b : résultats des tests ADCC**

| **Mutant** | **EC50 : Concentration d'anticorps (ng/ml) donnant 50 % de lyse maximale d'un contrôle interne** | **Ratio [WT]/[anticorps]** |
|---|---|---|
| WT | 0,45 | 1 |
| A3A-173 | 1,38 | 0,33 |
| K3B-90 | 0,75 | 0,60 |
| A3A-105 | 1,374 | 0,33 |
| A3A-31 | 1,839 | 0,24 |
| J3B-115 | 0,57 | 0,79 |

**Tableau 7c : résultats des tests ADCC (EC45 au lieu d'EC50)**

| **Mutant** | **EC45 : Concentration d'anticorps (ng/ml) donnant 45 % de lyse maximale d'un contrôle interne** | **Ratio [WT]/[anticorps]** |
|---|---|---|
| WT | 1,60 | 1 |
| O3A-44 | 2,6 | 0,62 |
| N3A-27 | 18,7 | 0,86 |
| N3A-58 | 4,4 | 0,36 |
| O3A-86 | 4,4 | 0,36 |
| N3A-87 | 1,0 | 1 |
| P4B-89 | 3,6 | 0,44 |
| O3A-05 | 0,2 | 8 |
| N3A-32 | 2,9 | 0,55 |
| Q3A-39 | 0,3 | 5,33 |
| Q4B-68 | 1,7 | 0,94 |

Les meilleurs variants sont A3A-184A et G3A-103.

### 4.5. Tests d'activité CDC

Des cellules cibles Raji exprimant l'antigène CD20 ont été incubées avec différentes concentrations d'anticorps anti-CD20 (0 à 5000 ng/ml) en présence de sérum de lapin comme source de complément (Cedarlane, dilution de 1/10). Après 1 heure d'incubation à 37°C, le niveau de LDH libérée dans le surnageant par les cellules cibles lysées a été mesuré chromogéniquement (kit de détection de cytotoxicité de Roche Applied Sciences) et utilisé pour quantifier la cytotoxicité dépendante du complément à médiation par les anticorps. Les résultats sont exprimés en pourcentage de lyse. L'EC50 (quantité d'anticorps qui induit 50% de lyse maximale) et Emax (pourcentage de lyse maximale) ont été calculés en utilisant le logiciel GraphPad PRISM.

Les résultats sont présentés en Tableaux 8 et 9.

**Tableau 8 : résultats des tests CDC**

| **Anticorps** | **Concentration d'anticorps (ng/ml) donnant 50 % de lyse d'un contrôle interne** | **Ratio [WT]/[anticorps]** |
|---|---|---|
| A3A-173 | 16,01 | 16.36 |
| K3B-90 | 23,12 | 11.33 |
| J3B-118 | 38,69 | 6.77 |
| A3A-105 | 64,76 | 4.05 |
| A3A-31 | 108,37 | 2.42 |
| J3B-115 | 145,44 | 1.80 |
| G3A-43 | 168,49 | 1.55 |
| G3A-45 | 195,20 | 1.34 |
| A3A-184 | 210,10 | 1.25 |
| J3B-16 | 210,10 | 1.25 |
| WT | 261,97 | 1 |

**Tableau 9a : résultats des tests CDC**

| **Anticorps** | **Concentration de l'anticorps (ng/ml) donnant 50 % de lyse d'un contrôle interne** | **Ratio [WT]/[anticorps]** |
|---|---|---|
| J3B-118A | 21,564 | 10.98 |
| J3A-28 | 31,004 | 7.64 |
| G3A-103 | 33,339 | 7.10 |
| A3A-105D | 38,550 | 6.14 |
| QL2A-16 | 51,544 | 4.59 |
| K3B-01 | 59,600 | 3.97 |
| J3A-06 | 64,089 | 6.70 |
| J3A-14 | 64,089 | 6.70 |
| A3A-184A | 74,107 | 3.20 |
| G3A-139 | 85,691 | 2.76 |
| J3B-23 | 99,085 | 2.39 |
| K3B-87 | 106,548 | 2.22 |
| A3A-34 | 123,202 | 1.92 |
| J3A-16 | 132,481 | 1.79 |
| DL4A-54 | 164,727 | 1.44 |
| G3A-95 | 177,134 | 1.34 |
| WT | 236,837 | 1 |
| G3A-88 | 273,856 | 0.86 |
| A3A-14 | 340,513 | 0.70 |
| A3A-90 | 566,098 | 0.42 |

**Tableau 9b : résultats des tests CDC**

| **Anticorps** | **Concentration de l'anticorps (ng/ml) donnant 50 % de lyse d'un contrôle interne** | **Ratio [WT]/[anticorps]** |
|---|---|---|
| WT | 133 | 1 |
| O3A-44 | 129 | 1,03 |
| N3A-27 | 179 | 0,74 |
| N3A-58 | 36 | 3,69 |
| O3A-86 | 44 | 3,02 |
| N3A-87 | 106 | 1,25 |
| P4B-89 | 115 | 1,16 |
| O3A-05 | 91 | 1,46 |
| N3A-32 | 66 | 2,02 |
| Q3A-39 | 61 | 2,18 |
| Q4B-68 | 126 | 1,06 |

Le tableau 10 suivant montre les résultats obtenus avec certains variants, classés par sous-groupe.

**Tableau 10**

| | | | | **Ratio ELISA (Ac purifiés)** | | | |
|---|---|---|---|---|---|---|---|
| **Nom** | **Mutations** | **ADCC** | **CDC** | **CD32aH** | **CD32aR** | **CD32b** | **CD64** |
| G3A-103 | K248E, A378V | 33,9 | 7,1 | 0,5 | 0,9 | 1,2 | 1,2 |
| J3A-28 | E333G, A378T, V397M | 18,4 | 7,6 | 1,1 | 1,8 | 1,6 | 1,3 |
| J3B-118A | P396L, N421T, A378V | 10,8 | 11,0 | 2,7 | 1,8 | 3,7 | 1,4 |
| J3B-118 | P396L, N421T | 9,7 | 12,2 | 1,0 | 1,1 | 1,0 | 1,6 |
| A3A-105D | G316D, K326E, A378V | 8,3 | 6,1 | 1,4 | 2,6 | 2,0 | 1,5 |
| A3A-14 | 5298N,A378V | 12,8 | 0,7 | 1,1 | 0,8 | 0,9 | 1,6 |
| G3A-95 | I336T, A378V | 10,8 | 1,3 | 0,7 | 0,9 | 1,0 | 1,3 |
| A3A-184A | K334N, P352S, V397M, A378V | 30,9 | 3,2 | 1,8 | 4,3 | 3,1 | 1,5 |
| J3B-23 | N286I, A378V, F423Y | 18,4 | 2,4 | ND | ND | ND | ND |
| K3B-01 | N315D, N361H, P396L | 15,2 | 4,0 | 5,7 | 1,0 | 1,0 | 2,3 |
| G3A-43 | A231V, A378V | 12,0 | 2,8 | 5,3 | 3,6 | 2,7 | 1,4 |
| J3A-06 | A378T, V397M, V412M | 8,3 | 3,7 | 0,84 | 2,0 | 2,4 | 1,3 |
| J3A-16 | N286Y, P352S, A378V | 8,3 | 1,8 | 2,61 | 1,7 | 1,7 | 1,3 |
| O3A-05 | K290E, T366A, A378V | 8,0 | 1,5 | 4,02 | 3,3 | 4,5 | 1,2 |
| Q3A-39 | N286I, P396L, N421T | 5,3 | 2,2 | 4,83 | 2,3 | 3,8 | 1,5 |
| A3A-184 | K334N, P352S, V397M | 5,3 | 2,2 | 1,33 | 3,2 | 3,3 | 1,4 |
| K3B-90 | V308A, K334R, A378V, K447N | 0,6 | 20,4 | 1,20 | 1,2 | 1,1 | 1,5 |
| A3A-173 | K248E, K334R, A378V | 0,3 | 29,5 | 0,95 | 1,3 | 1,3 | 1,2 |
| J3A-14 | K326T | 1,4 | 3,7 | 1,86 | 1,3 | 1,5 | 2,3 |
| G3A-88 | K320E, T394P, G402D | 1,3 | 0,9 | 2,94 | 3,7 | 3,9 | 2,4 |
| N3A-87 | K290E, K320E, T350A, P396L | 1,0 | 1,3 | 3,92 | 3,7 | 9,0 | 1,5 |
| J3B-115 | T359A, S383R, V397M | 0,8 | 3,2 | 4,65 | 5,1 | 4,2 | 1,5 |

Le tableau 11 suivant montre certains variants possibles selon l'invention :

**Tableau 11**

| **Nom** | **Mutations** | **Variant de départ** | **Mutation ajoutée** |
|---|---|---|---|
| G3A-95D | S298N, I336T, A378V | G3A-95 | S298N |
| K3B-90A | K248E, V308A, K334R, A378V, K447N | K3B-90 | K248E |
| G3A-88A | K320E, K326T, T394P, G402D | G3A-88 | K326T |
| N3A-87A | K290E, K320E, K326T, T350A, P396L | N3A-87 | K326T |
| A3A-105E | K248E, G316D, K326E, A378V | A3A-105D | K248E |
| A3A-14A | K248E, S298N, A378V | A3A-14 | K248E |
| A3A-184B | K248E, K334N, P352S, V397M, A378V | A3A-184 | K248E |
| G3A-43D | K248E, A231V, A378V | G3A-43 | K248E |
| G3A-95E | K248E, I336T, A378V | G3A-95 | K248E |
| J3A-28A | K248E, E333G, A378T, V397M | J3A-28 | K248E |
| J3B-118B | K248E, P396L, N421T, A378V | J3B-118A | K248E |
| J3B-23A | N286I, A378V, F423Y | J3B-23 | K248E |
| K3B-01A | N315D, N361H, P396L | K3B-01 | K248E |
| A3A-105F | G316D, K326E, E333G, A378V | A3A-105 | E333G |
| A3A-14B | S298N, E333G, A378V | A3A-14 | E333G |
| A3A-184C | E333G, K334N, P352S, V397M, A378V | A3A-184 | E333G |
| G3A-103A | K248E, E333G, A378V | G3A-103 | E333G |
| G3A-43E | A231V, E333G, A378V | G3A-43 | E333G |
| G3A-95F | E333G, I336T, A378V | G3A-95 | E333G |
| J3B-118C | E333G, P396L, N421T, A378V | J3B-118 | E333G |
| J3B-23B | N286I, E333G, A378V, F423Y | J3B-23 | E333G |
| K3B-01B | N315D, E333G, N361H, P396L | K3B-01 | E333G |
| A3A-105G | G316D, K326E, A378V, F423Y | A3A-105 | F423Y |
| A3A-14C | E333G, S298N, A378V, F423Y | A3A-14 | F423Y |
| A3A-184D | K334N, P352S, V397M, A378V, F423Y | A3A-184 | F423Y |
| G3A-103B | K248E, A378V, F423Y | G3A-103 | F423Y |
| G3A-43F | A231V, A378V, F423Y | G3A-43 | F423Y |
| G3A-95G | I336T, A378V, F423Y | G3A-95 | F423Y |
| J3A-28B | E333G, A378T, V397M, F423Y | J3A-28 | F423Y |
| J3B-118D | P396L, N421T, A378V, F423Y | J3B-118 | F423Y |
| K3B-01C | N315D, N361H, P396L,F423Y | K3B-01 | F423Y |

## Revendications

1. Méthode de production d'un polypeptide comprenant une région Fc d'IgG1 humaine mutée par rapport à un polypeptide parent et présentant une activité fonctionnelle médiée par la région Fc, qui est choisie parmi la cytotoxicité cellulaire dépendante des anticorps (ADCC) et la cytotoxicité dépendante du complément (CDC), augmentée par rapport à celle du polypeptide parent, le polypeptide parent comprenant une région Fc parente choisie parmi les séquences SEQ ID NO :1, 5, 6 et 10, ladite méthode comprenant une étape d'introduction d'une combinaison de mutations dans la région Fc parente choisie parmi :
248E/378V,
396L/421T/378 V,
316D/326E/378V,
298N/378V,
336T/378V,
334N/3 52 S/3 97M/378 V,
286I/378V/423Y,
231V/378V,
286Y/352S/378V,
290E/366A/378V,
308A/334R/378V/447N,
248E/334R/378V,
298N/336T/378V,
248E/308A/334R/378V/447N,
248E/316D/326E/378V,
248E/298N/378V,
248E/334N/352S/397M/378V,
248E/231V/378V,
248E/336T/378V,
248E/396L/421T/378 V,
316D/326E/333 G/378 V,
298N/333G/378V,
333G/334N/352S/397M/378V,
248E/333G/378V,
231V/333G/378V,
333G/336T/378V,
333G/396L/421T/378V,
286I/333G/378V/423Y,
316D/326E/378V/423Y,
333G/298N/378V/423Y,
334N/352S/397M/378V/423Y,
248E/378V/423Y,
231V/378V/423Y,
336T/378V/423Y, ou
396L/421T/378V/423Y,
la numérotation étant celle de l'index EU.

2. Méthode selon la revendication 1, comprenant une étape d'introduction d'une combinaison de mutations dans la région Fc parente choisie parmi
248E/378V,
396L/421T/378 V,
316D/326E/378V,
298N/378V,
336T/378V,
334N/3 52 S/3 97M/378 V,
286I/378V/423Y,
231V/378V,
286Y/352S/378V,
290E/366A/378V,
308A/334R/378V/447N, ou
248E/334R/378V.

3. Méthode selon l'une des revendications 1 à 2, **caractérisé en ce que** le polypeptide parent comprend une région Fc parente qui est la séquence SEQ ID NO :1.

4. Méthode selon l'une des revendications 1 à 3, **caractérisé en ce que** le polypeptide est choisi parmi une région Fc isolée, une séquence dérivée d'une région Fc isolée, un anticorps et une protéine de fusion comprenant une région Fc.

5. Méthode selon l'une des revendications 1 à 4, **caractérisé en ce que** le polypeptide est un polypeptide produit dans le lait d'animaux transgéniques.

6. Polypeptide consistant en la séquence SEQ ID NO :1 mutée selon l'une des combinaisons de mutations suivantes :
248E/378V,
396L/421T/378 V,
316D/326E/378V,
298N/378V,
336T/378V,
334N/3 52 S/3 97M/378 V,
286I/378V/423Y,
231V/378V,
286Y/352S/378V,
290E/366A/378V,
308A/334R/378V/447N,
248E/334R/378V,
298N/336T/378V,
248E/308A/334R/378V/447N,
248E/316D/326E/378V,
248E/298N/378V,
248E/334N/352S/397M/378V,
248E/231V/378V,
248E/336T/378V,
248E/396L/421T/378V,
316D/326E/333 G/378 V,
298N/333G/378V,
333G/334N/352S/397M/378V,
248E/333G/378V,
231V/333G/378V,
333G/336T/378V,
333G/396L/421T/378V,
286I/333G/378V/423Y,
316D/326E/378V/423Y,
333G/298N/378V/423Y,
334N/352S/397M/378V/423Y,
248E/378V/423Y,
231V/378V/423Y,
336T/378V/423Y, ou
396L/421T/378V/423Y,
la numérotation étant celle de l'index EU.

7. Composition pharmaceutique comprenant (i) un polypeptide selon la revendication 6, et (ii) au moins un excipient pharmaceutiquement acceptable.

8. Polypeptide selon la revendication 6, pour son utilisation comme médicament.

## Patentansprüche

1. Verfahren zur Herstellung eines Polypeptids, das eine im Verhältnis zu einem parentalen Polypeptid mutierte Fc-Region von menschlichem IgG1 umfasst und eine durch die Fc-Region vermittelte funktionelle Aktivität aufweist, die aus der antikörperabhängigen zellulären Zytotoxizität (ADCC) und der komplementabhängigen Zytotoxizität (CDC), die im Verhältnis zu der des parentalen Polypeptids erhöht ist, ausgewählt ist, wobei das parentale Polypeptid eine parentale Fc-Region umfasst, die aus den Sequenzen SEQ ID NO:1, 5, 6 und 10 ausgewählt ist, wobei das Verfahren einen Schritt des Einschleusens einer
Kombination von Mutationen in
die parentale Fc-Region umfasst, die ausgewählt ist aus:
248E/378V,
396L/421T/378V,
316D/326E/378V,
298N/378V,
336T/378V,
334N/352S/397M/378V,
286I/378V/423Y,
231V/378V,
286Y/352S/378V,
290E/366A/378V,
308A/334R/378V/447N,
248E/334R/378V,
298N/336T/378V,
248E/308A/334R/378V/447N,
248E/316D/326E/378V,
248E/298N/378V,
248E/334N/352S/397M/378V,
248E/231V/378V,
248E/336T/378V,
248E/396L/421T/378V,
316D/326E/333G/378V,
298N/333G/378V,
333G/334N/352S/397M/378V,
248E/333G/378V,
231V/333G/378V,
333G/336T/378V,
333G/396L/421T/378V,
286I/333G/378V/423Y,
316D/326E/378V/423Y,
333G/298N/378V/423Y,
334N/352S/397M/378V/423Y,
248E/378V/423Y,
231V/378V/423Y,
336T/378V/423Y oder
396L/421T/378V/423Y,
wobei die Nummerierung die des EU-Index ist.

2. Verfahren nach Anspruch 1, umfassend einen Schritt des Einschleusens einer
Kombination von Mutationen in die parentale Fc-Region, die ausgewählt ist aus:
248E/378V,
396L/421T/378V,
316D/326E/378V,
298N/378V,
336T/378V,
334N/352S/397M/378V,
286I/378V/423Y,
231V/378V,
286Y/352S/378V,
290E/366A/378V,
308A/334R/378V/447N oder
248E/334R/378V.

3. Verfahren nach einem der Ansprüche 1 bis 2, **dadurch gekennzeichnet, dass** das parentale Polypeptid eine parentale Fc-Region umfasst, bei der es sich um die Sequenz SEQ ID NO:1 handelt.

4. Verfahre nach einem der Ansprüche 1 bis 3, **dadurch gekennzeichnet, dass** das Polypeptid aus einer isolierten Fc-Region, einer Sequenz, die von einer isolierten Fc-Region abgeleitet ist, einem Antikörper und einem Fusionsprotein, das eine Fc-Region umfasst, ausgewählt ist.

5. Verfahren nach einem der Ansprüche 1 bis 4, **dadurch gekennzeichnet, dass** das Polypeptid ein Polypeptid ist, das in der Milch von transgenen Tieren produziert wird.

6. Polypeptid, bestehend aus der Sequenz SEQ ID NO:1, die gemäß einer der folgenden Mutationskombinationen mutiert ist:
248E/378V,
396L/421T/378V,
316D/326E/378V,
298N/378V,
336T/378V,
334N/352S/397M/378V,
286I/378V/423Y,
231V/378V,
286Y/352S/378V,
290E/366A/378V,
308A/334R/378V/447N,
248E/334R/378V,
298N/336T/378V,
248E/308A/334R/378V/447N,
248E/316D/326E/378V,
248E/298N/378V,
248E/334N/352S/397M/378V,
248E/231V/378V,
248E/336T/378V,
248E/396L/421T/378V,
316D/326E/333G/378V,
298N/333G/378V,
333G/334N/352S/397M/378V,
248E/333G/378V,
231V/333G/378V,
333G/336T/378V,
333G/396L/421T/378V,
286I/333G/378V/423Y,
316D/326E/378V/423Y,
333G/298N/378V/423Y,
334N/352S/397M/378V/423Y,
248E/378V/423Y,
231V/378V/423Y,
336T/378V/423Y oder
396L/421T/378V/423Y,
wobei die Nummerierung die des EU-Index ist.

7. Pharmazeutische Zusammensetzung, umfassend (i) ein Polypeptid nach Anspruch 6 und (ii) mindestens einen pharmazeutisch annehmbaren Hilfsstoff.

8. Polypeptid nach Anspruch 6, zur Verwendung als Arzneimittel.

## Claims

1. Method to produce a polypeptide comprising a human IgG1 Fc region that is mutated as compared to a parent polypeptide and having functional activity mediated by the Fc region that is selected from among antibody-dependent cell cytotoxicity (ADCC) and complement-dependent cytotoxicity (CDC), that is increased compared with that of the parent polypeptide, the parent polypeptide comprising a parent Fc region chosen from sequences SEQ ID NO:1, 5, 6 and 10, said method comprising a step to introduce a combination of mutations into the parent Fc region selected from among:
248E/378V,
396L/421T/378V,
316D/326E/378V,
298N/378V,
336T/378V,
334N/352S/397M/378V,
286I/378V/423Y,
231V/378V,
286Y/352S/378V,
290E/366A/378V,
308A/334R/378V/447N,
248E/334R/378V,
298N/336T/378V,
248E/308A/334R/378V/447N,
248E/316D/326E/378V,
248E/298N/378V,
248E/334N/352S/397M/378V,
248E/231V/378V,
248E/336T/378V,
248E/396L/421T/378V,
316D/326E/333G/378V,
298N/333G/378V,
333G/334N/352S/397M/378V,
248E/333G/378V,
231V/333G/378V,
333G/336T/378V,
333G/396L/421T/378V,
286I/333G/378V/423Y,
316D/326E/378V/423Y,
333G/298N/378V/423Y,
334N/352S/397M/378V/423Y,
248E/378V/423Y,
231V/378V/423Y,
336T/378V/423Y, or
396L/421T/378V/423Y,
the numbering being that of the EU Index.

2. Method according to claim 1, comprising a step to introduce a combination of mutations into the parent Fc region selected from among:
248E/378V,
396L/421T/378V,
316D/326E/378V,
298N/378V,
336T/378V,
334N/352S/397M/378V,
286I/378V/423Y,
231V/378V,
286Y/352S/378V,
290E/366A/378V,
308A/334R/378V/447N, or
248E/334R/378V.

3. Method according to claim 1 or 2, **characterized in that** the parent polypeptide comprises a parent Fc region that is sequence SEQ ID NO:1.

4. Method according to one of claims 1 to 3, **characterized in that** the polypeptide is selected from among an isolated Fc region, a sequence derived from an isolated Fc region, an antibody and a fusion protein comprising an Fc region.

5. Method according to one of claims 1 to 4, **characterized in that** the polypeptide is a polypeptide produced in the milk of transgenic animals.

6. Polypeptide consisting in sequence SEQ ID NO:1 that is mutated according to one of the following combinations:
248E/378V,
396L/421T/378V,
316D/326E/378V,
298N/378V,
336T/378V,
334N/352S/397M/378V,
286I/378V/423Y,
231V/378V,
286Y/352S/378V,
290E/366A/378V,
308A/334R/378V/447N,
248E/334R/378V,
298N/336T/378V,
248E/308A/334R/378V/447N,
248E/316D/326E/378V,
248E/298N/378V,
248E/334N/352S/397M/378V,
248E/231V/378V,
248E/336T/378V,
248E/396L/421T/378V,
316D/326E/333G/378V,
298N/333G/378V,
333G/334N/352S/397M/378V,
248E/333G/378V,
231V/333G/378V,
333G/336T/378V,
333G/396L/421T/378V,
286I/333G/378V/423Y,
316D/326E/378V/423Y,
333G/298N/378V/423Y,
334N/352S/397M/378V/423Y,
248E/378V/423Y,
231V/378V/423Y,
336T/378V/423Y, or
396L/421T/378V/423Y,
the numbering being that of the EU Index.

7. Pharmaceutical composition comprising (i) a polypeptide according to claim 6, and (ii) at least one pharmaceutically acceptable excipient.

8. Polypeptide according to claim 6, for use as medicinal product.
